# EUROPEAN PATENT SPECIFICATION

(11) **EP 1 706 493 B1**
(45) Date of publication and mention of the grant of the patent: **02.05.2012**
(21) Application number: 05701017.5
(22) Date of filing: 18.01.2005
(51) Int. Cl.: C12P 13/08

(54) **PROCESS FOR PRODUCING L-THREONINE OR L-LYSINE EMPLOYING RECOMBINANT ENTEROBACTERIACEAE WITH ENHANCED ENOLASE ACTIVITY**
VERFAHREN ZUR HERSTELLUNG VON L-THREONIN ODER L-LYSIN UNTER VERWENDUNG REKOMBINANTER ENTEROBACTERIACEAE MIT ERHÖHTER ENOLASEAKTIVITÄT
PROCÉDÉ DE PRÉPARATION DE L-THRÉONINE OU DE L-LYSINE EN UTILISANT DES ENTEROBACTERIACEAE RECOMBINANTES À L'ACTIVITÉ D'ÉNOLASE AMÉLIORÉE

(30) Priority: 23.01.2004 DE 102004003410
(43) Date of publication of application: 04.10.2006
(73) Proprietor: Evonik Degussa GmbH, 45128 Essen (DE)
(72) Inventor: RIEPING, Mechthild, 33619 Bielefeld (DE)
(86) International application number: PCT/EP2005/000448
(87) International publication number: WO 2005/071094

(56) References cited:
- EP-A- 0 877 090
- EP-A- 1 352 966
- EP-A- 1 382 685
- WO-A-01/04325
- WO-A-03/023016
- DATABASE EMBL [Online] 1 August 1988 (1988-08-01), BLATTNER, F.R. ET AL.: "Enolase (EC 4.2.1.11) (2-phosphoglycerate dehydratase) (2-phospho-D-glycerate hydro-lyase)" XP002334858 Database accession no. P0A6P9 cited in the application
- DATABASE EMBL [Online] 1 August 1988 (1988-08-01), JIN, Q. ET AL.: "Enolase (EC 4.2.1.11) (2-phosphoglycerate dehydratase) (2-phospho-D-glycerate hydro-lyase)" XP002334859 Database accession no. P0A6Q2 cited in the application
- DATABASE EMBL [Online] 28 February 2003 (2003-02-28), MCCLELLAND, M. ET AL.: "Enolase (EC 4.2.1.11) (2-phosphoglycerate dehydratase) (2-phospho-D-glycerate hydro-lyase)" XP002334860 Database accession no. Q8XGP6 cited in the application

### Sequence listings

<110> Degussa AG
<120> Process for the preparation of L-threonine or L-lysine using strains of the Enterobacteriaceae family
<130> 020479 BT
<160> 8
<170> PatentIn version 3.2
<210> 1
   <211> 31
   <212> DNA
   <213> Artificial sequence
<220>
   <221> Primer
   <222> (1)..(31)
   <223> eno1
<400> 1
   gtttgtctag agtttcagtt taactagtga c 31
<210> 2
   <211> 25
   <212> DNA
   <213> Artificial sequence
<220>
   <221> Primer
   <222> (1)..(25)
   <223> eno2
<400> 2
   ccggaggctg gcaagcttaa atcag 25
<210> 3
   <211> 1381
   <212> DNA
   <213> Escherichia coli
<220>
   <221> PCR product
   <222> (1)..(1381)
<220>
   <221> CDS
   <222> (46)..(1344)
   <223> eno gene
<400> 3
   gtttgtctag agtttcagtt taactagtga cttgaggaaa accta atg tcc aaa atc      57
                                                     Met Ser Lys Ile
                                                     1
   gta aaa atc atc ggt cgt gaa atc atc gac tcc cgt ggt aac ccg act      105
   Val Lys Ile Ile Gly Arg Glu Ile Ile Asp Ser Arg Gly Asn Pro Thr
   5                   10                  15                  20
   gtt gaa gcc gaa gta cat ctg gag ggt ggt ttc gtc ggt atg gca gct      153
   Val Glu Ala Glu Val His Leu Glu Gly Gly Phe Val Gly Met Ala Ala
                   25                  30                  35
   gct ccg tca ggt gct tct act ggt tcc cgt gaa gct ctg gaa ctg cgc      201
   Ala Pro Ser Gly Ala Ser Thr Gly Ser Arg Glu Ala Leu Glu Leu Arg
               40                  45                  50
   gat ggc gac aaa tcc cgt ttc ctg ggt aaa ggc gta acc aaa gct gtt      249
   Asp Gly Asp Lys Ser Arg Phe Leu Gly Lys Gly Val Thr Lys Ala Val
           55                  60                  65
   gct gcg gta aac ggc ccg atc gct cag gcg ctg att ggc aaa gat gct      297
   Ala Ala Val Asn Gly Pro Ile Ala Gln Ala Leu Ile Gly Lys Asp Ala
       70                  75                  80
   aaa gat cag gct ggc att gac aag atc atg atc gac ctg gac ggc acc      345
   Lys Asp Gln Ala Gly Ile Asp Lys Ile Met Ile Asp Leu Asp Gly Thr
   85                  90                  95                  100
   gaa aac aaa tcc aaa ttc ggc gcg aac gca atc ctg gct gta tct ctg      393
   Glu Asn Lys Ser Lys Phe Gly Ala Asn Ala Ile Leu Ala Val Ser Leu
                   105                 110                 115
   gct aac gcc aaa gct gct gca gct gct aaa ggt atg ccg ctg tac gag      441
   Ala Asn Ala Lys Ala Ala Ala Ala Ala Lys Gly Met Pro Leu Tyr Glu
               120                 125                 130
   cac atc gct gaa ctg aac ggt act ccg ggc aaa tac tct atg ccg gtt      489
   His Ile Ala Glu Leu Asn Gly Thr Pro Gly Lys Tyr Ser Met Pro Val
           135                 140                 145
   ccg atg atg aac atc atc aac ggt ggt gag cac gct gac aac aac gtt      537
   Pro Met Met Asn Ile Ile Asn Gly Gly Glu His Ala Asp Asn Asn Val
       150                 155                 160
   gat atc cag gaa ttc atg att cag ccg gtt ggc gcg aaa act gtg aaa      585
   Asp Ile Gln Glu Phe Met Ile Gln Pro Val Gly Ala Lys Thr Val Lys
   165                 170                 175                 180
   gaa gcc atc cgc atg ggt tct gaa gtt ttc cat cac ctg gca aaa gtt      633
   Glu Ala Ile Arg Met Gly Ser Glu Val Phe His His Leu Ala Lys Val
                   185                 190                 195
   ctg aaa gcg aaa ggc atg aac act gct gtt ggt gac gaa ggt ggc tat      681
   Leu Lys Ala Lys Gly Met Asn Thr Ala Val Gly Asp Glu Gly Gly Tyr
               200                 205                 210
   gcg ccg aac ctg ggt tcc aac gct gaa gct ctg gct gtt atc gct gaa      729
   Ala Pro Asn Leu Gly Ser Asn Ala Glu Ala Leu Ala Val Ile Ala Glu
           215                 220                 225
   gct gtt aaa gct gct ggt tat gaa ctg ggc aaa gac atc act ttg gcg      777
   Ala Val Lys Ala Ala Gly Tyr Glu Leu Gly Lys Asp Ile Thr Leu Ala
       230                 235                 240
   atg gac tgc gca gct tct gaa ttc tac aaa gat ggt aaa tac gtt ctg      825
   Met Asp Cys Ala Ala Ser Glu Phe Tyr Lys Asp Gly Lys Tyr Val Leu
   245                 250                 255                 260
   gct ggc gaa ggc aac aaa gcg ttc acc tct gaa gaa ttc act cac ttc      873
   Ala Gly Glu Gly Asn Lys Ala Phe Thr Ser Glu Glu Phe Thr His Phe
                   265                 270                 275
   ctg gaa gaa ctg acc aaa cag tac ccg atc gtt tct atc gaa gac ggt      921
   Leu Glu Glu Leu Thr Lys Gln Tyr Pro Ile Val Ser Ile Glu Asp Gly
               280                 285                 290
   ctg gac gaa tct gac tgg gac ggt ttc gca tac cag acc aaa gtt ctg      969
   Leu Asp Glu Ser Asp Trp Asp Gly Phe Ala Tyr Gln Thr Lys Val Leu
           295                 300                 305
   ggc gac aaa atc cag ctg gtt ggt gac gac ctg ttc gta acc aac acc     1017
   Gly Asp Lys Ile Gln Leu Val Gly Asp Asp Leu Phe Val Thr Asn Thr
       310                 315                 320
   aag atc ctg aaa gaa ggt atc gaa aaa ggt atc gct aac tcc atc ctg     1065
   Lys Ile Leu Lys Glu Gly Ile Glu Lys Gly Ile Ala Asn Ser Ile Leu
   325                 330                 335                 340
   atc aaa ttc aac cag atc ggt tct ctg acc gaa act ctg gct gca atc     1113
   Ile Lys Phe Asn Gln Ile Gly Ser Leu Thr Glu Thr Leu Ala Ala Ile
                   345                 350                 355
   aag atg gcg aaa gat gct ggc tac act gca gtt atc tct cac cgt tct     1161
   Lys Met Ala Lys Asp Ala Gly Tyr Thr Ala Val Ile Ser His Arg Ser
               360                 365                 370
   ggc gaa act gaa gac gct acc atc gct gac ctg gct gtt ggt act gct     1209
   Gly Glu Thr Glu Asp Ala Thr Ile Ala Asp Leu Ala Val Gly Thr Ala
           375                 380                 385
   gca ggc cag atc aaa act ggt tct atg agc cgt tct gac cgt gtt gct     1257
   Ala Gly Gln Ile Lys Thr Gly Ser Met Ser Arg Ser Asp Arg Val Ala
       390                 395                 400
   aaa tac aac cag ctg att cgt atc gaa gaa gct ctg ggc gaa aaa gca     1305
   Lys Tyr Asn Gln Leu Ile Arg Ile Glu Glu Ala Leu Gly Glu Lys Ala
   405                 410                 415                 420
   ccg tac aac ggt cgt aaa gag atc aaa ggc cag gca taa gactgacttt      1354
   Pro Tyr Asn Gly Arg Lys Glu Ile Lys Gly Gln Ala
                   425                 430
   atctgattta agcttgccag cctccgg                                       1381
<210> 4
   <211> 432
   <212> PRT
   <213> Escherichia coli
<400> 4
   Met Ser Lys Ile Val Lys Ile Ile Gly Arg Glu Ile Ile Asp Ser Arg
   1               5                   10                  15
   Gly Asn Pro Thr Val Glu Ala Glu Val His Leu Glu Gly Gly Phe Val
               20                  25                  30
   Gly Met Ala Ala Ala Pro Ser Gly Ala Ser Thr Gly Ser Arg Glu Ala
           35                  40                  45
   Leu Glu Leu Arg Asp Gly Asp Lys Ser Arg Phe Leu Gly Lys Gly Val
       50                  55                  60
   Thr Lys Ala Val Ala Ala Val Asn Gly Pro Ile Ala Gln Ala Leu Ile
   65                  70                  75                  80
   Gly Lys Asp Ala Lys Asp Gln Ala Gly Ile Asp Lys Ile Met Ile Asp
                   85                  90                  95
   Leu Asp Gly Thr Glu Asn Lys Ser Lys Phe Gly Ala Asn Ala Ile Leu
               100                 105                 110
   Ala Val Ser Leu Ala Asn Ala Lys Ala Ala Ala Ala Ala Lys Gly Met
           115                 120                 125
   Pro Leu Tyr Glu His Ile Ala Glu Leu Asn Gly Thr Pro Gly Lys Tyr
       130                 135                 140
   Ser Met Pro Val Pro Met Met Asn Ile Ile Asn Gly Gly Glu His Ala
   145                 150                 155                 160
   Asp Asn Asn Val Asp Ile Gln Glu Phe Met Ile Gln Pro Val Gly Ala
                   165                 170                 175
   Lys Thr Val Lys Glu Ala Ile Arg Met Gly Ser Glu Val Phe His His
               180                 185                 190
   Leu Ala Lys Val Leu Lys Ala Lys Gly Met Asn Thr Ala Val Gly Asp
           195                 200                 205
   Glu Gly Gly Tyr Ala Pro Asn Leu Gly Ser Asn Ala Glu Ala Leu Ala
       210                 215                 220
   Val Ile Ala Glu Ala Val Lys Ala Ala Gly Tyr Glu Leu Gly Lys Asp
   225                 230                 235                 240
   Ile Thr Leu Ala Met Asp Cys Ala Ala Ser Glu Phe Tyr Lys Asp Gly
                   245                 250                 255
   Lys Tyr Val Leu Ala Gly Glu Gly Asn Lys Ala Phe Thr Ser Glu Glu
               260                 265                 270
   Phe Thr His Phe Leu Glu Glu Leu Thr Lys Gln Tyr Pro Ile Val Ser
           275                 280                 285
   Ile Glu Asp Gly Leu Asp Glu Ser Asp Trp Asp Gly Phe Ala Tyr Gln
       290                 295                 300
   Thr Lys Val Leu Gly Asp Lys Ile Gln Leu Val Gly Asp Asp Leu Phe
   305                 310                 315                 320
   Val Thr Asn Thr Lys Ile Leu Lys Glu Gly Ile Glu Lys Gly Ile Ala
                   325                 330                 335
   Asn Ser Ile Leu Ile Lys Phe Asn Gln Ile Gly Ser Leu Thr Glu Thr
               340                 345                 350
   Leu Ala Ala Ile Lys Met Ala Lys Asp Ala Gly Tyr Thr Ala Val Ile
           355                 360                 365
   Ser His Arg Ser Gly Glu Thr Glu Asp Ala Thr Ile Ala Asp Leu Ala
       370                 375                 380
   Val Gly Thr Ala Ala Gly Gln Ile Lys Thr Gly Ser Met Ser Arg Ser
   385                 390                 395                 400
   Asp Arg Val Ala Lys Tyr Asn Gln Leu Ile Arg Ile Glu Glu Ala Leu
                   405                 410                 415
   Gly Glu Lys Ala Pro Tyr Asn Gly Arg Lys Glu Ile Lys Gly Gln Ala
   420 425 430
<210> 5
   <211> 1299
   <212> DNA
   <213> Shigella flexneri
<220>
   <221> CDS
   <222> (1)..(1299)
   <223> eno coding region
<400> 5
   atg tcc aaa atc gta aaa atc atc ggt cgt gaa atc atc gac tcc cgt      48
   Met Ser Lys Ile Val Lys Ile Ile Gly Arg Glu Ile Ile Asp Ser Arg
   1               5                   10                  15
   ggt aac ccg act gtt gaa gcc gaa gta cat ctg gag ggt ggt ttc gtc      96
   Gly Asn Pro Thr Val Glu Ala Glu Val His Leu Glu Gly Gly Phe Val
               20                  25                  30
   ggt atg gca gct gct ccg tca ggt gct tct act ggt tcc cgt gaa gct      144
   Gly Met Ala Ala Ala Pro Ser Gly Ala Ser Thr Gly Ser Arg Glu Ala
           35                  40                  45
   ctg gaa ctg cgc gat ggc gac aaa tcc cgt ttc ctg ggt aaa ggc gta      192
   Leu Glu Leu Arg Asp Gly Asp Lys Ser Arg Phe Leu Gly Lys Gly Val
       50                  55                  60
   acc aaa gct gtt gct gcg gta aac ggc ccg atc gct cag gcg ctg att      240
   Thr Lys Ala Val Ala Ala Val Asn Gly Pro Ile Ala Gln Ala Leu Ile
   65                  70                  75                  80
   ggc aaa gat gct aaa gat cag gct ggc att gac aag atc atg atc gac      288
   Gly Lys Asp Ala Lys Asp Gln Ala Gly Ile Asp Lys Ile Met Ile Asp
                   85                  90                  95
   ctg gac ggc acc gaa aac aaa tcc aaa ttc ggc gcg aac gca atc ctg      336
   Leu Asp Gly Thr Glu Asn Lys Ser Lys Phe Gly Ala Asn Ala Ile Leu
               100                 105                 110
   gct gta tct ctg gct aac gcc aaa gct gct gca gct gct aaa ggt atg      384
   Ala Val Ser Leu Ala Asn Ala Lys Ala Ala Ala Ala Ala Lys Gly Met
           115                 120                 125
   ccg ctg tac gag cac atc gct gaa ctg aac ggt act ccg ggc aaa tac      432
   Pro Leu Tyr Glu His Ile Ala Glu Leu Asn Gly Thr Pro Gly Lys Tyr
       130                 135                 140
   tcc atg ccg gtt ccg atg atg aac atc atc aac ggt ggt gag cac gct      480
   Ser Met Pro Val Pro Met Met Asn Ile Ile Asn Gly Gly Glu His Ala
   145                 150                 155                 160
   gac aac aac gtt gat atc cag gaa ttc atg att cag ccg gtt ggc gcg      528
   Asp Asn Asn Val Asp Ile Gln Glu Phe Met Ile Gln Pro Val Gly Ala
                   165                 170                 175
   aaa act gtg aaa gaa gcc atc cgc atg ggt tct gaa gtt ttc cat cac      576
   Lys Thr Val Lys Glu Ala Ile Arg Met Gly Ser Glu Val Phe His His
               180                 185                 190
   ctg gca aaa gtt ctg aaa gcg aaa ggc atg aac act gct gtt ggt gac      624
   Leu Ala Lys Val Leu Lys Ala Lys Gly Met Asn Thr Ala Val Gly Asp
           195                 200                 205
   gaa ggt ggc tat gcg ccg aac ctg ggt tcc aac gct gaa gct ctg gct      672
   Glu Gly Gly Tyr Ala Pro Asn Leu Gly Ser Asn Ala Glu Ala Leu Ala
       210                 215                 220
   gtt atc gct gaa gct gtt aaa gct gct ggt tat gaa ctg ggc aaa gac      720
   Val Ile Ala Glu Ala Val Lys Ala Ala Gly Tyr Glu Leu Gly Lys Asp
   225                 230                 235                 240
   atc act ctg gcg atg gac tgc gca gct tct gaa ttc tac aaa gat ggt      768
   Ile Thr Leu Ala Met Asp Cys Ala Ala Ser Glu Phe Tyr Lys Asp Gly
                   245                 250                 255
   aaa tac gtt ctg gct ggc gaa ggc aac aaa gcg ttc acc tct gaa gaa      816
   Lys Tyr Val Leu Ala Gly Glu Gly Asn Lys Ala Phe Thr Ser Glu Glu
               260                 265                 270
   ttc act cac ttc ctg gaa gaa ctg acc aaa cag tac ccg atc gtt tct      864
   Phe Thr His Phe Leu Glu Glu Leu Thr Lys Gln Tyr Pro Ile Val Ser
           275                 280                 285
   atc gaa gac ggt ctg gac gaa tct gac tgg gac ggt ttc gca tac cag      912
   Ile Glu Asp Gly Leu Asp Glu Ser Asp Trp Asp Gly Phe Ala Tyr Gln
       290                 295                 300
   acc aaa gtt ctg ggc gac aaa atc cag ctg gtt ggt gac gac ctg ttc      960
   Thr Lys Val Leu Gly Asp Lys Ile Gln Leu Val Gly Asp Asp Leu Phe
   305                 310                 315                 320
   gta acc aac acc aag atc ctg aaa gaa ggt atc gaa aaa ggt atc gct     1008
   Val Thr Asn Thr Lys Ile Leu Lys Glu Gly Ile Glu Lys Gly Ile Ala
                   325                 330                 335
   aac tcc atc ctg atc aaa ttc aac cag atc ggt tct ctg acc gaa act     1056
   Asn Ser Ile Leu Ile Lys Phe Asn Gln Ile Gly Ser Leu Thr Glu Thr
               340                 345                 350
   ctg gct gca atc aag atg gcg aaa gat gct ggc tac act gca gtt atc     1104
   Leu Ala Ala Ile Lys Met Ala Lys Asp Ala Gly Tyr Thr Ala Val Ile
           355                 360                 365
   tct cac cgt tct ggt gaa act gaa gat gct acc atc gct gat ctg gct     1152
   Ser His Arg Ser Gly Glu Thr Glu Asp Ala Thr Ile Ala Asp Leu Ala
       370                 375                 380
   gtt ggt act gct gca ggc cag atc aaa act ggt tct atg agc cgt tct     1200
   Val Gly Thr Ala Ala Gly Gln Ile Lys Thr Gly Ser Met Ser Arg Ser
   385                 390                 395                 400
   gac cgt gtt gct aaa tac aac cag ctg att cgt atc gaa gaa gct ctg     1248
   Asp Arg Val Ala Lys Tyr Asn Gln Leu Ile Arg Ile Glu Glu Ala Leu
                   405                 410                 415
   ggc gaa aaa gca ccg tac aac ggt cgt aaa gag atc aaa ggc cag gca     1296
   Gly Glu Lys Ala Pro Tyr Asn Gly Arg Lys Glu Ile Lys Gly Gln Ala
               420                 425                 430
   taa                                                                 1299
<210> 6
   <211> 432
   <212> PRT
   <213> Shigella flexneri
<400> 6
   Met Ser Lys Ile Val Lys Ile Ile Gly Arg Glu Ile Ile Asp Ser Arg
   1               5                   10                  15
   Gly Asn Pro Thr Val Glu Ala Glu Val His Leu Glu Gly Gly Phe Val
               20                  25                  30
   Gly Met Ala Ala Ala Pro Ser Gly Ala Ser Thr Gly Ser Arg Glu Ala
           35                  40                  45
   Leu Glu Leu Arg Asp Gly Asp Lys Ser Arg Phe Leu Gly Lys Gly Val
       50                  55                  60
   Thr Lys Ala Val Ala Ala Val Asn Gly Pro Ile Ala Gln Ala Leu Ile
   65                  70                  75                  80
   Gly Lys Asp Ala Lys Asp Gln Ala Gly Ile Asp Lys Ile Met Ile Asp
                   85                  90                  95
   Leu Asp Gly Thr Glu Asn Lys Ser Lys Phe Gly Ala Asn Ala Ile Leu
               100                 105                 110
   Ala Val Ser Leu Ala Asn Ala Lys Ala Ala Ala Ala Ala Lys Gly Met
           115                 120                 125
   Pro Leu Tyr Glu His Ile Ala Glu Leu Asn Gly Thr Pro Gly Lys Tyr
       130                 135                 140
   Ser Met Pro Val Pro Met Met Asn Ile Ile Asn Gly Gly Glu His Ala
   145                 150                 155                 160
   Asp Asn Asn Val Asp Ile Gln Glu Phe Met Ile Gln Pro Val Gly Ala
                   165                 170                 175
   Lys Thr Val Lys Glu Ala Ile Arg Met Gly Ser Glu Val Phe His His
               180                 185                 190
   Leu Ala Lys Val Leu Lys Ala Lys Gly Met Asn Thr Ala Val Gly Asp
           195                 200                 205
   Glu Gly Gly Tyr Ala Pro Asn Leu Gly Ser Asn Ala Glu Ala Leu Ala
       210                 215                 220
   Val Ile Ala Glu Ala Val Lys Ala Ala Gly Tyr Glu Leu Gly Lys Asp
   225                 230                 235                 240
   Ile Thr Leu Ala Met Asp Cys Ala Ala Ser Glu Phe Tyr Lys Asp Gly
                   245                 250                 255
   Lys Tyr Val Leu Ala Gly Glu Gly Asn Lys Ala Phe Thr Ser Glu Glu
               260                 265                 270
   Phe Thr His Phe Leu Glu Glu Leu Thr Lys Gln Tyr Pro Ile Val Ser
           275                 280                 285
   Ile Glu Asp Gly Leu Asp Glu Ser Asp Trp Asp Gly Phe Ala Tyr Gln
       290                 295                 300
   Thr Lys Val Leu Gly Asp Lys Ile Gln Leu Val Gly Asp Asp Leu Phe
   305                 310                 315                 320
   Val Thr Asn Thr Lys Ile Leu Lys Glu Gly Ile Glu Lys Gly Ile Ala
   325 330 335
   Asn Ser Ile Leu Ile Lys Phe Asn Gln Ile Gly Ser Leu Thr Glu Thr
   340 345 350
   Leu Ala Ala Ile Lys Met Ala Lys Asp Ala Gly Tyr Thr Ala Val Ile
   355 360 365
   Ser His Arg Ser Gly Glu Thr Glu Asp Ala Thr Ile Ala Asp Leu Ala
   370 375 380
   Val Gly Thr Ala Ala Gly Gln Ile Lys Thr Gly Ser Met Ser Arg Ser
   385 390 395 400
   Asp Arg Val Ala Lys Tyr Asn Gln Leu Ile Arg Ile Glu Glu Ala Leu
   405 410 415
   Gly Glu Lys Ala Pro Tyr Asn Gly Arg Lys Glu Ile Lys Gly Gln Ala
   420 425 430
<210> 7
   <211> 1299
   <212> DNA
   <213> Salmonella typhimurium
<220>
   <221> CDS
   <222> (1)..(1299)
   <223> eno coding region
<400> 7
   atg tcc aaa atc gtt aaa gtc atc ggt cgt gaa atc atc gac tcc cgt      48
   Met Ser Lys Ile Val Lys Val Ile Gly Arg Glu Ile Ile Asp Ser Arg
   1               5                   10                  15
   ggt aac ccg act gtt gaa gct gaa gta cac ctg gaa ggt ggt ttc gta      96
   Gly Asn Pro Thr Val Glu Ala Glu Val His Leu Glu Gly Gly Phe Val
               20                  25                  30
   ggt atg gcg gcg gct ccg tca ggt gct tct act ggt tcc cgc gaa gcg     144
   Gly Met Ala Ala Ala Pro Ser Gly Ala Ser Thr Gly Ser Arg Glu Ala
           35                  40                  45
   ctg gaa ctg cgc gat ggc gac aaa tcc cgt ttc ctg ggt aaa ggc gta     192
   Leu Glu Leu Arg Asp Gly Asp Lys Ser Arg Phe Leu Gly Lys Gly Val
       50                  55                  60
   acc aaa gct gtt ggc gcg gtt aac ggc ccg atc gct cag gct att ctt     240
   Thr Lys Ala Val Gly Ala Val Asn Gly Pro Ile Ala Gln Ala Ile Leu
   65                  70                  75                  80
   ggc aaa gac gct aaa gac cag gct ggc atc gac aaa atc atg atc gac     288
   Gly Lys Asp Ala Lys Asp Gln Ala Gly Ile Asp Lys Ile Met Ile Asp
                   85                  90                  95
   ctg gac ggt act gaa aac aaa tct aac ttc ggt gca aac gcc att ctg     336
   Leu Asp Gly Thr Glu Asn Lys Ser Asn Phe Gly Ala Asn Ala Ile Leu
               100                 105                 110
   gct gtc tct ctg gct aac gcc aaa gct gct gct gcc gct aaa ggt atg     384
   Ala Val Ser Leu Ala Asn Ala Lys Ala Ala Ala Ala Ala Lys Gly Met
           115                 120                 125
   ccg ctg tac gag cac att gct gaa ctg aat ggc acg ccg ggc aaa tac     432
   Pro Leu Tyr Glu His Ile Ala Glu Leu Asn Gly Thr Pro Gly Lys Tyr
           130                 135                 140
   tcc atg ccg gtt ccg atg atg aac atc atc aac ggc ggc gag cac gct      480
   Ser Met Pro Val Pro Met Met Asn Ile Ile Asn Gly Gly Glu His Ala
   145                 150                 155                 160
   gac aac aac gtc gac atc cag gaa ttc atg atc cag ccg gtt ggc gcg      528
   Asp Asn Asn Val Asp Ile Gln Glu Phe Met Ile Gln Pro Val Gly Ala
                   165                 170                 175
   aaa acg gtt aaa gaa gcc atc cgt atg ggt tct gaa gtt ttc cat cac      576
   Lys Thr Val Lys Glu Ala Ile Arg Met Gly Ser Glu Val Phe His His
               180                 185                 190
   ctg gca aaa gtg ctg aaa ggc aaa ggc atg aac acc gct gtg ggt gac      624
   Leu Ala Lys Val Leu Lys Gly Lys Gly Met Asn Thr Ala Val Gly Asp
           195                 200                 205
   gaa ggc ggc tat gcg ccg aac ctg ggt tcc aac gct gaa gcg ctg gct      672
   Glu Gly Gly Tyr Ala Pro Asn Leu Gly Ser Asn Ala Glu Ala Leu Ala
       210                 215                 220
   gtt atc gct gaa gcg gtt aaa gcg gct ggt tac gag ctg ggt aaa gac      720
   Val Ile Ala Glu Ala Val Lys Ala Ala Gly Tyr Glu Leu Gly Lys Asp
   225                 230                 235                 240
   atc acc ctg gcg atg gac tgc gca gca tct gaa ttc tac aaa gac ggt      768
   Ile Thr Leu Ala Met Asp Cys Ala Ala Ser Glu Phe Tyr Lys Asp Gly
                   245                 250                 255
   aaa tac gtt ctg gct ggc gaa ggc aac aaa gcg ttc acc tcc gaa gaa      816
   Lys Tyr Val Leu Ala Gly Glu Gly Asn Lys Ala Phe Thr Ser Glu Glu
               260                 265                 270
   ttc acc cac ttc ctg gaa gaa ctg acc aaa cag tac ccg atc gtt tcc      864
   Phe Thr His Phe Leu Glu Glu Leu Thr Lys Gln Tyr Pro Ile Val Ser
           275                 280                 285
   atc gaa gat ggt ctg gac gag tct gac tgg gac ggt ttt gca tac cag      912
   Ile Glu Asp Gly Leu Asp Glu Ser Asp Trp Asp Gly Phe Ala Tyr Gln
       290                 295                 300
   acc aaa gta ctg ggc gac aaa atc cag ctg gtt ggt gac gac ctg ttc      960
   Thr Lys Val Leu Gly Asp Lys Ile Gln Leu Val Gly Asp Asp Leu Phe
   305                 310                 315                 320
   gta acc aac acc aaa atc ctg aaa gaa ggc atc gag aaa ggc atc gct      1008
   Val Thr Asn Thr Lys Ile Leu Lys Glu Gly Ile Glu Lys Gly Ile Ala
                   325                 330                 335
   aac tcc atc ctg atc aaa ttc aac cag atc ggt tct ctg acc gaa act      1056
   Asn Ser Ile Leu Ile Lys Phe Asn Gln Ile Gly Ser Leu Thr Glu Thr
               340                 345                 350
   ctg gct gca atc aag atg gcg aaa gac gct ggc tat act gct gtc atc      1104
   Leu Ala Ala Ile Lys Met Ala Lys Asp Ala Gly Tyr Thr Ala Val Ile
           355                 360                 365
   tct cac cgt tct ggc gaa act gaa gac gct acc atc gct gac ctg gct      1152
   Ser His Arg Ser Gly Glu Thr Glu Asp Ala Thr Ile Ala Asp Leu Ala
       370                 375                 380
   gtt ggt acc gct gca ggc cag atc aaa acc ggt tct atg agc cgt tct      1200
   Val Gly Thr Ala Ala Gly Gln Ile Lys Thr Gly Ser Met Ser Arg Ser
   385                 390                 395                 400
   gac cgt gtt gct aaa tac aac cag ctg att cgt atc gaa gaa gct ctg      1248
   Asp Arg Val Ala Lys Tyr Asn Gln Leu Ile Arg Ile Glu Glu Ala Leu
                   405                 410                 415
   ggc gaa aaa gca ccg tat aac ggt cgt aaa gag atc aaa ggc cag gcg      1296
   Gly Glu Lys Ala Pro Tyr Asn Gly Arg Lys Glu Ile Lys Gly Gln Ala
               420                 425                 430
   taa                                                                  1299
<210> 8
   <211> 432
   <212> PRT
   <213> Salmonella typhimurium
<400> 8
   Met Ser Lys Ile Val Lys Val Ile Gly Arg Glu Ile Ile Asp Ser Arg
   1               5                   10                  15
   Gly Asn Pro Thr Val Glu Ala Glu Val His Leu Glu Gly Gly Phe Val
               20                  25                  30
   Gly Met Ala Ala Ala Pro Ser Gly Ala Ser Thr Gly Ser Arg Glu Ala
           35                  40                  45
   Leu Glu Leu Arg Asp Gly Asp Lys Ser Arg Phe Leu Gly Lys Gly Val
       50                  55                  60
   Thr Lys Ala Val Gly Ala Val Asn Gly Pro Ile Ala Gln Ala Ile Leu
   65                  70                  75                  80
   Gly Lys Asp Ala Lys Asp Gln Ala Gly Ile Asp Lys Ile Met Ile Asp
                   85                  90                  95
   Leu Asp Gly Thr Glu Asn Lys Ser Asn Phe Gly Ala Asn Ala Ile Leu
               100                 105                 110
   Ala Val Ser Leu Ala Asn Ala Lys Ala Ala Ala Ala Ala Lys Gly Met
           115                 120                 125
   Pro Leu Tyr Glu His Ile Ala Glu Leu Asn Gly Thr Pro Gly Lys Tyr
       130                 135                 140
   Ser Met Pro Val Pro Met Met Asn Ile Ile Asn Gly Gly Glu His Ala
   145                 150                 155                 160
   Asp Asn Asn Val Asp Ile Gln Glu Phe Met Ile Gln Pro Val Gly Ala
                   165                 170                 175
   Lys Thr Val Lys Glu Ala Ile Arg Met Gly Ser Glu Val Phe His His
               180                 185                 190
   Leu Ala Lys Val Leu Lys Gly Lys Gly Met Asn Thr Ala Val Gly Asp
           195                 200                 205
   Glu Gly Gly Tyr Ala Pro Asn Leu Gly Ser Asn Ala Glu Ala Leu Ala
       210                 215                 220
   Val Ile Ala Glu Ala Val Lys Ala Ala Gly Tyr Glu Leu Gly Lys Asp
   225                 230                 235                 240
   Ile Thr Leu Ala Met Asp Cys Ala Ala Ser Glu Phe Tyr Lys Asp Gly
                   245                 250                 255
   Lys Tyr Val Leu Ala Gly Glu Gly Asn Lys Ala Phe Thr Ser Glu Glu
               260                 265                 270
   Phe Thr His Phe Leu Glu Glu Leu Thr Lys Gln Tyr Pro Ile Val Ser
           275                 280                 285
   Ile Glu Asp Gly Leu Asp Glu Ser Asp Trp Asp Gly Phe Ala Tyr Gln
       290                 295                 300
   Thr Lys Val Leu Gly Asp Lys Ile Gln Leu Val Gly Asp Asp Leu Phe
   305                 310                 315                 320
   Val Thr Asn Thr Lys Ile Leu Lys Glu Gly Ile Glu Lys Gly Ile Ala
                   325                 330                 335
   Asn Ser Ile Leu Ile Lys Phe Asn Gln Ile Gly Ser Leu Thr Glu Thr
               340                 345                 350
   Leu Ala Ala Ile Lys Met Ala Lys Asp Ala Gly Tyr Thr Ala Val Ile
           355                 360                 365
   Ser His Arg Ser Gly Glu Thr Glu Asp Ala Thr Ile Ala Asp Leu Ala
       370                 375                 380
   Val Gly Thr Ala Ala Gly Gln Ile Lys Thr Gly Ser Met Ser Arg Ser
   385                 390                 395                 400
   Asp Arg Val Ala Lys Tyr Asn Gln Leu Ile Arg Ile Glu Glu Ala Leu
                   405                 410                 415
   Gly Glu Lys Ala Pro Tyr Asn Gly Arg Lys Glu Ile Lys Gly Gln Ala
               420                 425                 430

## Description

### Field of the Invention

This invention relates to a process for the preparation of L-threonine and L-lysine, using recombinant microorganisms of the Enterobacteriaceae family in which the eno gene is enhanced, in particular over-expressed, and to these microorganisms.

### Background of the Invention

L-Amino acids, in particular L-threonine, are used in human medicine and in the pharmaceuticals industry, in the foodstuffs industry and very particularly in animal nutrition.

It is known that L-amino acids are prepared by fermentation of strains of Enterobacteriaceae, in particular Escherichia coli (E. coli) and Serratia marcescens. Because of their great importance, work is constantly being undertaken to improve the preparation processes. Improvements to the process can relate to fermentation measures, such as e.g. stirring and supply of oxygen, or the composition of the nutrient media, such as e.g. the sugar concentration during the fermentation, or the working up to the product form, by e.g. ion exchange chromatography, or the intrinsic output properties of the microorganism itself.

Methods of mutagenesis, selection and mutant selection are used to improve the output properties of these microorganisms. Strains which are resistant to antimetabolites, such as e.g. the threonine analogue α-amino-β-hydroxyvaleric acid (AHV), or are auxotrophic for metabolites of regulatory importance and produce L-amino acid, such as e.g. L-threonine, are obtained in this manner.

Methods of the recombinant DNA technique have also been employed for some years for improving the strain of strains of the Enterobacteriaceae family which produce L-amino acids, by amplifying individual amino acid biosynthesis genes and investigating the effect on the production. Summarizing information on the cell and molecular biology of Escherichia coli and Salmonella are to be found in Neidhardt (ed): Escherichia coli and Salmonella, Cellular and Molecular Biology, 2nd Edition, ASM Press, Washington, D.C., USA (1996).

The patent document EP 1 382 685 A2 (DEGUSSA AG, 21.01.2004) discloses a process for the fermentative production of L-threonine employing recombinant microorganisms of the Enterobacteriaceae family, wherein the L-threonine productivity of an *Escherichia coli* strain has been improved by enhancing the RseB protein activity.

### Object of the Invention

The object of the invention is to provide new measures for improved fermentative preparation of L-threonine and L-lysine.

### Summary of the Invention

Disclosed are recombinant microorganisms of the Enterobacteriaceae family which contain an enhanced or over-expressed eno gene, which codes for enolase, or alleles thereof, and which produce L-threonine and L-lysine, in an improved manner.

The starting point for the comparison are in each case the microorganisms which are not recombinant for the eno gene and contain no enhanced eno gene.

These microorganisms include, in particular, microorganisms of the Enterobacteriaceae family in which a polynucleotide which codes for a polypeptide of which the amino acid sequence is identical to the extent of at least 90%, in particular to the extent of at least 95%, preferably to the extent of at least 98% or at least 99%, particularly preferably to the extent of 99.7% and very particularly preferably to the extent of 100% to an amino acid sequence chosen from the group consisting of SEQ ID No. 4, SEQ ID No. 6, and SEQ ID No. 8 is enhanced.

Amino acid sequences which are identical to those from SEQ ID No. 4, SEQ ID No. 6 or SEQ ID No. 8 are preferred.

The microorganisms mentioned contain enhanced or over-expressed polynucleotides chosen from the group consisting of:
a) polynucleotide with the nucleotide sequence SEQ ID No. 3, SEQ ID No. 5 or SEQ ID No. 7;
b) polynucleotide with a nucleotide sequence which corresponds to SEQ ID No. 3, SEQ ID No. 5 or SEQ ID No. 7 within the context of degeneration of the genetic code;
c) polynucleotide sequence with a sequence which hybridizes under stringent conditions with the sequence complementary to SEQ ID No. 3, SEQ ID No. 5 or SEQ ID No. 7;
d) polynucleotide with a sequence SEQ ID No. 3, SEQ ID No. 5 or SEQ ID No. 7 which contains neutral-function sense mutants,
where the polynucleotides code for enolase.

The invention provides a process for the fermentative preparation of L-threonine and L-lysine, using recombinant microorganisms of the Enterobacteriaceae family which, in particular, already produce the L-amino acids and in which at least the eno gene or nucleotide sequences which code for the gene product thereof is or are enhanced.

### Detailed Description of the Invention

The microorganisms described are preferably employed.

Where L-amino acids or amino acids are mentioned in the following, this means one or more amino acids, including their salts, chosen from the group consisting of L-threonine and L-lysine, L-Threonine is particularly preferred.

The term "enhancement" in this connection describes the increase in the intracellular activity or concentration of one or more enzymes or proteins in a microorganism which are coded by the corresponding DNA, for example by increasing the number of copies of the gene or genes by at least one (1) copy, linking a potent promoter functionally to the gene or using a gene or allele which codes for a corresponding enzyme or protein with a high activity, and optionally combining these measures.

Alleles are in general understood as meaning alternative forms of a given gene. The forms are distinguished by differences in the nucleotide sequence.

The protein coded by a nucleotide sequence, i.e. an ORF, a gene or an allele, or the ribonucleic acid coded is in general called the gene product.

By enhancement measures the activity or concentration of the corresponding protein is in general increased by at least 10%, 25%, 50%, 75%, 100%, 150%, 200%, 300%, 400% or 500%, up to a maximum of 1,000% or 2,000%, based on that of the wild-type protein or on the activity or concentration of the protein in the microorganism or parent strain which is not recombinant for the corresponding enzyme or protein. Microorganism or parent strain which is not recombinant is understood as meaning the microorganism on which the measures are carried out.

The invention provides a process for the preparation of L-threonine or L-lysine by fermentation of recombinant microorganisms of the Enterobacteriaceae family, characterized in that
a) the microorganisms of the Enterobacteriaceae family which produce the desired L-amino acid and in which the eno gene or alleles thereof is/are enhanced, in particular over-expressed, are cultured in a medium under conditions suitable for the formation of the eno gene product,
b) the desired L-amino acid is concentrated in the medium or in the cells of the microorganisms, and
c) the desired L-amino acid is isolated, constituents of the fermentation broth and/or the biomass in its entirety or portions (≥ 0 to 100%) thereof optionally remaining in the product which has been isolated or being completely removed.

The recombinant microorganisms with an enhanced eno gene can produce L-amino acids from glucose, sucrose, lactose, fructose, maltose, molasses, optionally starch, optionally cellulose or from glycerol and ethanol. They are representatives of the Enterobacteriaceae family chosen from the genera Escherichia, Erwinia, Providencia and Serratia. The genera Escherichia and Serratia are preferred. Of the genus Escherichia in particular the species Escherichia coli and of the genus Serratia in particular the species Serratia marcescens are to be mentioned.

Recombinant microorganisms are in general produced by transformation, transduction or conjugation, or a combination of these methods, with a vector which contains the desired gene, an allele of this gene or parts thereof and/or a promoter which enhances the expression of the gene. This promoter can be the promoter originating by enhancing mutation from the endogenous regulatory sequence upstream of the gene, or an efficient promoter that has been fused with the gene.

Suitable strains, which produce L-threonine in particular, of the genus Escherichia, in particular of the species Escherichia coli, are, for example
- Escherichia coli H4581 (EP 0 301 572)
- Escherichia coli KY10935 (Bioscience Biotechnology and Biochemistry 61(11):1877-1882 (1997)
- Escherichia coli VNIIgenetika MG442 (US-A-4278,765)
- Escherichia coli VNIIgenetika M1 (US-A-4,321,325)
- Escherichia coli VNIIgenetika 472T23 (US-A-5,631,157)
- Escherichia coli BKIIM B-3996 (US-A-5,175,107)
- Escherichia coli kat 13 (WO 98/04715)
- Escherichia coli KCCM-10132 (WO 00/09660).

Suitable L-threonine-producing strains of the genus Serratia, in particular of the species Serratia marcescens, are, for example
- Serratia marcescens HNr21 (Applied and Environmental Microbiology 38(6): 1045-1051 (1979))
- Serratia marcescens TLr156 (Gene 57(2-3): 151-158 (1987))
- Serratia marcescens T-2000 (Applied Biochemistry and Biotechnology 37(3): 255-265 (1992)).

Strains from the Enterobacteriaceae family which produce L-threonine preferably have, inter alia, one or more genetic or phenotypic features chosen from the group consisting of: resistance to α-amino-β-hydroxyvaleric acid, resistance to thialysine, resistance to ethionine, resistance to α-methylserine, resistance to diaminosuccinic acid, resistance to α-aminobutyric acid, resistance to borrelidin, resistance to cyclopentane-carboxylic acid, resistance to rifampicin, resistance to valine analogues, such as, for example, valine hydroxamate, resistance to purine analogues, such as, for example, 6-dimethylaminopurine, a need for L-methionine, optionally a partial and compensatable need for L-isoleucine, a need for meso-diaminopimelic acid, auxotrophy in respect of threonine-containing dipeptides, resistance to L-threonine resistance to threonine raffinate, resistance to L-homoserine, resistance to L-lysine, resistance to L-methionine, resistance to L-glutamic acid, resistance to L-aspartate, resistance to L-leucine, resistance to L-phenylalanine, resistance to L-serine, resistance to L-cysteine, resistance to L-valine, sensitivity to fluoropyruvate, defective threonine dehydrogenase, optionally an ability for sucrose utilization, enhancement of the threonine operon, enhancement of homoserine dehydrogenase I-aspartate kinase I, preferably of the feed back resistant form, enhancement of homoserine kinase, enhancement of threonine synthase, enhancement of aspartate kinase, optionally of the feed back resistant form, enhancement of aspartate semialdehyde dehydrogenase, enhancement of phosphoenol pyruvate carboxylase, optionally of the feed back resistant form, enhancement of phosphoenol pyruvate synthase, enhancement of transhydrogenase, enhancement of the RhtB gene product, enhancement of the RhtC gene product, enhancement of the YfiK gene product, enhancement of a pyruvate carboxylase, and attenuation of acetic acid formation.

It has been found that microorganisms of the Enterobacteriaceae family produce L-threonine and L-lysine, in an improved manner after enhancement, in particular over-expression, of the eno gene or alleles thereof.

The nucleotide sequences of the genes of Escherichia coli belong to the prior art (see the following text references) and can also be found in the genome sequence of Escherichia coli published by Blattner et al. (Science 277: 1453-1462 (1997)). It is known that enzymes endogenous in the host (methionine aminopeptidase) can split off the N-terminal amino acid methionine.

The nucleotide sequences for the eno gene are likewise known from Shigella flexneri and Salmonella typhimurium, which also belong to the Enterobacteriaceae family.

The eno gene is described, inter alia, by the following data:

| | |
|---|---|
| Description: Function: | enolase, phosphopyruvate hydratase enolization (splitting off of water): 2-phosphoglycerate to phosphoenol pyruvate in glycolysis |
| EC No.: | EC 4.2.1.11 |
| Reference: | Spring TG. and Wold F.; The Journal of Biological Chemistry 246(22): 6797-6802 (1971) |
| | Klein et al.; DNA Sequence 6(6): 351-355 (1996) |
| | Gulick et al.; Biochemistry 40(51): 15716 -15724 (2001) |
| | Kaga et al.; Bioscience, Biotechnology and Biochemistry 66(10): 2216-2220 (2002) |
| Accession No.: | AE000361 |

The eno gene from Salmonella typhimurium is described, inter alia, in the following reference: Garrido-Pertierra A.; Revista Espanola de Fisiologia 36(1): 33-39 (1980).

The nucleic acid sequences can be found in the databanks of the National Center for Biotechnology Information (NCBI) of the National Library of Medicine (Bethesda, MD, USA), the nucleotide sequence databank of the European Molecular Biologies Laboratories (EMBL, Heidelberg, Germany or Cambridge, UK) or the DNA databank of Japan (DDBJ, Mishima, Japan).

For better clarity, the known nucleotide sequence of the eno gene from Escherichia coli is to be found in SEQ ID No. 3 and the known sequences for the eno gene from Shigella flexneri (AE015293) and Salmonella typhimurium (AE008835) are to be found under SEQ ID No. 5 or SEQ ID No. 7. The amino acid sequences of the proteins coded by these reading frames are shown as SEQ ID No. 4, SEQ ID No. 6 or SEQ ID No. 8.

The genes described in the text references mentioned can be used according to the invention. Alleles of the genes which result from the degeneracy of the genetic code or due to "sense mutations" of neutral function can furthermore be used. The use of endogenous genes is preferred.

"Endogenous genes" or "endogenous nucleotide sequences" are understood as meaning the genes or alleles or nucleotide sequences present in the population of a species.

Suitable alleles of the eno gene which contain neutral-function sense mutations include, inter alia, those which lead to at most 50 or to at most 40 or to at most 30 or to at most 20, preferably to at most 10 or to at most 5, very particularly preferably to at most 3 or to at most 2 or to at least one (1) conservative amino acid exchange in the protein coded by them.

In the case of aromatic amino acids, conservative exchanges are referred to when phenylalanine, tryptophan and tyrosine are exchanged for one another. In the case of hydrophobic amino acids, conservative exchanges are referred to when leucine, isoleucine and valine are exchanged for one another. In the case of polar amino acids, conservative exchanges are referred to when glutamine and asparagine are exchanged for one another. In the case of basic amino acids, conservative exchanges are referred to when arginine, lysine and histidine are exchanged for one another. In the case of acidic amino acids, conservative exchanges are referred to when aspartic acid and glutamic acid are exchanged for one another. In the case of amino acids containing hydroxyl groups, conservative exchanges are referred to when serine and threonine are exchanged for one another. All other amino acid exchanges are called non-conservative amino acid exchanges.

In the same way, those nucleotide sequences which code for variants of the proteins mentioned which additionally contain a lengthening or shortening by at least one (1) amino acid on the N or C terminus can also be used. This lengthening or shortening is not more than 50, 40, 30, 20, 10, 5, 3 or 2 amino acids or amino acid radicals.

Suitable alleles also include those which code for proteins in which at least one (1) amino acid is inserted (insertion) or removed (deletion). The maximum number of such changes, called indels, can relate to 2, 3, 5, 10, 20 but in no case more than 30 amino acids.

Suitable alleles furthermore include those which are obtainable by hybridization, in particular under stringent conditions, using SEQ ID No. 3, SEQ ID No. 5 or SEQ ID No. 7 or parts thereof, in particular the coding regions or the sequences complementary thereto.

Instructions for identifying DNA sequences by means of hybridization can be found by the expert, inter alia, in the handbook "The DIG System Users Guide for Filter Hybridization" from Boehringer Mannheim GmbH (Mannheim, Germany, 1993) and in Liebl et al. (International Journal of Systematic Bacteriology 41: 255-260 (1991)). The hybridization takes place under stringent conditions, that is to say only hybrids in which the probe and target sequence, i.e. the polynucleotides treated with the probe, are at least 80% identical are formed. It is known that the stringency of the hybridization, including the washing steps, is influenced or determined by varying the buffer composition, the temperature and the salt concentration. The hybridization reaction is in general carried out under a relatively low stringency compared with the washing steps (Hybaid Hybridisation Guide, Hybaid Limited, Teddington, UK, 1996).

A buffer corresponding to 5x SSC buffer at a temperature of approx. 50ºC - 68ºC, for example, can be employed for the hybridization reaction. Probes can also hybridize here with polynucleotides which are less than 80% identical to the sequence of the probe. Such hybrids are less stable and are removed by washing under stringent conditions. This can be achieved, for example, by lowering the salt concentration to 2x SSC and optionally subsequently 0.5x SSC (The DIG System User's Guide for Filter Hybridisation, Boehringer Mannheim, Mannheim, Germany, 1995) a temperature of approx. 50ºC - 68ºC, approx. 52ºC - 68ºC, approx. 54ºC - 68ºC, approx. 56ºC - 68ºC, approx. 58ºC - 68ºC, approx. 60ºC - 68ºC, approx. 62 - 68ºC, approx. 64ºC - 68ºC, approx. 66ºC - 68ºC being established. Temperature ranges of approx. 64ºC - 68ºC or approx. 66ºC - 68ºC are preferred. It is optionally possible to lower the salt concentration to a concentration corresponding to 0.2x SSC or 0.1x SSC. Polynucleotide fragments which are, for example, at least 80% or at least 90%, at least 91%, at least 92%, at least 93%, at least 94%, at least 95%, at least 96%, at least 97%, at least 98% or at least 99% identical to the sequence of the probe employed or to the nucleotide sequences shown in SEQ ID No. 3, SEQ ID No. 5 or SEQ ID No. 7 can be isolated by increasing the hybridization temperature stepwise from 50ºC to 68ºC in steps of approx. 1 - 2ºC. Further instructions on hybridization are obtainable on the market in the form of so-called kits (e.g. DIG Easy Hyb from Roche Diagnostics GmbH, Mannheim, Germany, Catalogue No. 1603558). The nucleotide sequences obtained in this way code for polypeptides which are at least 90% identical, in particular identical to the extent of at least 95%, preferably to the extent of at least 98% or at least 99%, very particularly preferably to the extent of 99.7% to the amino acid sequences shown in SEQ ID No. 4, SEQ ID No. 6 or SEQ ID No. 8.

To achieve an enhancement, for example, expression of the genes or the catalytic properties of the enzyme proteins can be increased. The two measures can optionally be combined.

Thus, for example, the number of copies of the corresponding genes can be increased, or the promoter and regulation region or the ribosome binding site upstream of the structural gene can be mutated. Expression cassettes which are incorporated upstream of the structural gene act in the same way. By inducible promoters, it is additionally possible to increase the expression in the course of fermentative L-threonine production, and it may furthermore be advantageous also to use promoters for the gene expression which allow another gene expression with respect to time. The expression is likewise improved by measures to prolong the life of the m-RNA. Furthermore, the enzyme activity is also increased by preventing the degradation of the enzyme protein. The genes or gene constructs can either be present in plasmids with a varying number of copies, or can be integrated and amplified in the chromosome. Alternatively, an over-expression of the genes in question can furthermore be achieved by changing the composition of the media and the culture procedure.

Methods of overexpression are adequately described in the prior art - for example by Makrides et al. (Microbiological Reviews 60 (3), 512-538 (1996)). By using vectors, the number of copies is increased by at least one (1) copy. Vectors which can be used are plasmids such as are described, for example, in US 5,538,873. Vectors which can also be used are phages, for example the phage Mu, as described in EP 0332448, or the phage lambda (λ). An increase in the number of copies can also be achieved by incorporating a further copy into a further site of the chromosome - for example into the att site of the phage λ (Yu and Court, Gene 223, 77-81 (1998)). US 5,939,307 discloses that by incorporation of expression cassettes or promoters, such as, for example, the tac promoter, trp promoter, lpp promoter or P_{L} promoter and P_{R}-promoter of the phage λ for example upstream of the chromosomal threonine operon it was possible to achieve an increase in the expression. The promoters of the phage T7, the gear-box promoters or the nar promoter can be used in the same way. Such expression cassettes or promoters can also be used, as described in EP 0 593 792, to overexpress plasmid-bound genes. By using the lacI^{Q} allele, the expression of plasmid-bound genes can in turn be controlled (Glascock and Weickert, Gene 223, 221-231 (1998)). It is furthermore possible that the activity of the promoters is increased by modification of their sequence by means of one or more nucleotide exchanges, by insertion(s) and/or deletion(s). Another gene expression with respect to time can be achieved, for example, as described by Walker et al. (Journal of Bacteriology 181: 1269-80 (1999)) by using the growth phase-dependent fis promoter.

The expert can find general instructions in this respect, inter alia, in Chang and Cohen (Journal of Bacteriology 134: 1141-1156 (1978)), in Hartley and Gregori (Gene 13: 347-353 (1981)), in Amann and Brosius (Gene 40: 183-190 (1985)), in de Broer et al. (Proceedings of the National Academy of Sciences of the United States of America 80: 21-25 (1983)), in LaVallie et al. (BIO/TECHNOLOGY 11: 187-193 (1993)), in PCT/US97/13359, in Llosa et al. (Plasmid 26: 222-224 (1991)), in Quandt and Klipp (Gene 80: 161-169 (1989)), in Hamilton et al. (Journal of Bacteriology 171: 4617-4622 (1989)), in Jensen and Hammer (Biotechnology and Bioengineering 58: 191-195 (1998)) and in known textbooks of genetics and molecular biology.

Plasmid vectors which can replicate in Enterobacteriaceae, such as e.g. cloning vectors derived from pACYC184 (Bartolomé et al.; Gene 102: 75-78 (1991)), pTrc99A (Amann et al.; Gene 69: 301-315 (1988)) or pSC101 derivatives (Vocke and Bastia; Proceedings of the National Academy of Sciences USA 80(21): 6557-6561 (1983)) can be used. A strain transformed with a plasmid vector, where the plasmid vector carries at least the eno gene or a nucleotide sequence which codes for the gene product thereof or an allele, can be employed in a process according to the invention.

The term transformation is understood as meaning the uptake of an isolated nucleic acid by a host (microorganism).

It is also possible to transfer mutations which affect the expression of the particular genes into various strains by sequence exchange (Hamilton et al.; (Journal of Bacteriology 171: 4617-4622 (1989)), conjugation or transduction.

More detailed explanations of the terms in genetics and molecular biology are found in known textbooks of genetics and molecular biology, such as, for example, the textbook by Birge (Bacterial and Bacteriophage Genetics, 4th ed., Springer Verlag, New York (USA), 2000) or the textbook by Berg, Tymoczko and Stryer (Biochemistry, 5th ed., Freeman and Company, New York (USA), 2002) or the handbook by Sambrook et al. (Molecular Cloning, A Laboratory Manual, (3 volume set), Cold Spring Harbor Laboratory Press, Cold Spring Harbor (USA), 2001).

It may furthermore be advantageous for the production of L-threonine with strains of the Enterobacteriaceae family to enhance one or more enzymes of the known threonine biosynthesis pathway or enzymes of anaplerotic metabolism or enzymes for the production of reduced nicotinamide adenine dinucleotide phosphate or enzymes of glycolysis or PTS enzymes or enzymes of sulfur metabolism, in addition to the enhancement of the eno gene. The use of endogenous genes is in general preferred.

Thus, for example, at the same time one or more of the genes chosen from the group consisting of
- at least one gene of the thrABC operon which codes for aspartate kinase, homoserine dehydrogenase, homoserine kinase and threonine synthase (US-A-4,278,765),
- the pyc gene of Corynebacterium glutamicum which codes for pyruvate carboxylase (WO 99/18228),
- the pps gene which codes for phosphoenol pyruvate synthase (Molecular and General Genetics 231(2): 332-336 (1992)),
- the ppc gene which codes for phosphoenol pyruvate carboxylase (WO 02/064808),
- the pntA and pntB genes which code for subunits of pyridine transhydrogenase (European Journal of Biochemistry 158: 647-653 (1986)),
- the rhtB gene which codes for the protein which imparts homoserine resistance (EP-A-O 994 190),
- the rhtC gene which codes for the protein which imparts threonine resistance
   (EP-A-1 013 765),
- the thrE gene of Corynebacterium glutamicum which codes for the threonine export carrier protein (WO 01/92545),
- the gdhA gene which codes for glutamate dehydrogenase (Nucleic Acids Research 11: 5257-5266 (1983); Gene 23: 199-209 (1983)),
- the pgm gene which codes for phosphoglucomutase (WO 03/004598),
- the fba gene which codes for fructose biphosphate aldolase (WO 03/004664),
- the ptsH gene of the ptsHIcrr operon which codes for the phosphohistidine protein hexose phosphotransferase of the phosphotransferase system PTS (WO 03/004674),
- the ptsI gene of the ptsHIcrr operon which codes for enzyme I of the phosphotransferase system PTS (WO 03/004674),
- the crr gene of the ptsHIcrr operon which codes for the glucose-specific IIA component of the phosphotransferase system PTS (WO 03/004674),
- the ptsG gene which codes for the glucose-specific IIBC component (WO 03/004670),
- the lrp gene which codes for the regulator of the leucine regulon (WO 03/004665),
- the fadR gene which codes for the regulator of the fad regulon (WO 03/038106),
- the iclR gene which codes for the regulator of central intermediate metabolism (WO 03/038106),
- the ahpC gene of the ahpCF operon which codes for the small sub-unit of alkyl hydroperoxide reductase (WO 03/004663),
- the ahpF gene of the ahpCF operon which codes for the large sub-unit of alkyl hydroperoxide reductase (WO 03/004663),
- the cysK gene which codes for cysteine synthase A (WO 03/006666),
- the cysB gene which codes for the regulator of the cys regulon (WO 03/006666),
- the cysJ gene of the cysJIH operon which codes for the flavoprotein of NADPH sulfite reductase (WO 03/006666),
- the cysI gene of the cysJIH operon which codes for the haemoprotein of NADPH sulfite reductase (WO 03/006666),
- the cysH gene of the cysJIH operon which codes for adenylyl sulfate reductase (WO 03/006666),
- the rseA gene of the rseABC operon which codes for a membrane protein with anti-sigmaE activity (WO 03/008612),
- the rseC gene of the rseABC operon which codes for a global regulator of the sigmaE factor (WO 03/008612),
- the sucA gene of the sucABCD operon which codes for the decarboxylase sub-unit of 2-ketoglutarate dehydrogenase (WO 03/008614),
- the sucB gene of the sucABCD operon which codes for the dihydrolipoyltranssuccinase E2 sub-unit of 2-ketoglutarate dehydrogenase (WO 03/008614),
- the sucC gene of the sucABCD operon which codes for the β-sub-unit of succinyl-CoA synthetase (WO 03/008615),
- the sucD gene of the sucABCD operon which codes for the α-sub-unit of succinyl-CoA synthetase (WO 03/008615),
- the aceE gene which codes for the E1 component of the pyruvate dehydrogenase complex (WO 03/076635),
- the aceF gene which codes for the E2 component of the pyruvate dehydrogenase complex (WO 03/076635), and
- the rseB gene which codes for the regulator of sigmaE factor activity (Molecular Microbiology 24(2): 355-371 (1997)),
- the gene product of the open reading frame (ORF) yodA of Escherichia coli (Accession Number AE000288 of the National Center for Biotechnology Information (NCBI, Bethesda, MD, USA), DE10361192.4),
- the gene product of the open reading frame (ORF) yaaU of Escherichia coli (Accession Number AE005181 of the National Center for Biotechnology Information (NCBI, Bethesda, MD, USA), DE10361268.8) and
- the malT gene which codes for the positive transcriptional activator of the maltose regulon (Gene 42: 201-208 (1986)
can be enhanced.

It may furthermore be advantageous for the production of L-threonine in addition to the enhancement of the eno gene, for one or more of the genes chosen from the group consisting of
- the tdh gene which codes for threonine dehydrogenase (Journal of Bacteriology 169: 4716-4721 (1987)),
- the mdh gene which codes for malate dehydrogenase (E.C. 1.1.1.37) (Archives in Microbiology 149: 36-42 (1987)),
- the gene product of the open reading frame (orf) yjfA of Escherichia coli (Accession Number AAC77180 of the National Center for Biotechnology Information (NCBI,
   Bethesda, MD, USA),
   WO 02/29080)),
- the gene product of the open reading frame (orf) ytfP of Escherichia coli (Accession Number AAC77179 of the National Center for Biotechnology Information (NCBI, Bethesda, MD, USA), WO 02/29080)),
- the pckA gene which codes for the enzyme phosphoenol pyruvate carboxykinase (WO 02/29080),
- the poxB gene which codes for pyruvate oxidase (WO 02/36797),
- the dgsA gene which codes for the DgsA regulator of the phosphotransferase system (WO 02/081721) and is also known under the name of the mlc gene,
- the fruR gene which codes for the fructose repressor (WO 02/081698) and is also known under the name of the cra gene,
- the rpoS gene which codes for the sigma³⁸ factor (WO 01/05939) and is also known under the name of the katF gene, and
- the aspA gene which codes for aspartate ammonium lyase (WO 03/008603)
to be attenuated, in particular eliminated or for the expression thereof to be reduced.

The term "attenuation" in this connection describes the reduction or elimination of the intracellular activity or concentration of one or more enzymes or proteins in a microorganism which are coded by the corresponding DNA, for example by using a weaker promoter than in the microorganism or parent strain which is not recombinant for the corresponding enzyme or protein or a gene or allele which codes for a corresponding enzyme or protein with a low activity or inactivating the corresponding enzyme (protein) or the open reading frame or the gene and optionally combining these measures.

By attenuation measures, the activity or concentration of the corresponding protein is in general reduced to 0 to 75%, 0 to 50%, 0 to 25%, 0 to 10% or 0 to 5% of the activity or concentration of the wild-type protein or of the activity or concentration of the protein in the microorganism or parent strain which is not recombinant for the corresponding enzyme or protein. Microorganism or parent strain which is not recombinant is understood as meaning the microorganism on which the measures are carried out.

To achieve an attenuation, for example, expression of the genes or open reading frames or the catalytic properties of the enzyme proteins can be reduced or eliminated. The two measures can optionally be combined.

The reduction in gene expression can take place by suitable culturing, by genetic modification (mutation) of the signal structures of gene expression or also by the antisense-RNA technique. Signal structures of gene expression are, for example, repressor genes, activator genes, operators, promoters, attenuators, ribosome binding sites, the start codon and terminators. The expert can find information in this respect, inter alia, for example, in Jensen and Hammer (Biotechnology and Bioengineering 58: 191-195 (1998)), in Carrier und Keasling (Biotechnology Progress 15: 58-64 (1999)), Franch and Gerdes (Current Opinion in Microbiology 3: 159-164 (2000)) and in known textbooks of genetics and molecular biology, such as, for example, the textbook by Knippers ("Molekulare Genetik [Molecular Genetics]", 6th edition, Georg Thieme Verlag, Stuttgart, Germany, 1995) or that by Winnacker ("Gene und Klone [Genes and Clones]", VCH Verlagsgesellschaft, Weinheim, Germany, 1990).

Mutations which lead to a change or reduction in the catalytic properties of enzyme proteins are known from the prior art. Examples which may be mentioned are the works of Qiu and Goodman (Journal of Biological Chemistry 272: 8611-8617 (1997)), Yano et al. (Proceedings of the National Academy of Sciences of the United States of America 95: 5511-5515 (1998)), Wente and Schachmann (Journal of Biological Chemistry 266: 20833-20839 (1991)). Summarizing descriptions can be found in known textbooks of genetics and molecular biology, such as e.g. that by Hagemann ("Allgemeine Genetik [General Genetics]", Gustav Fischer Verlag, Stuttgart, 1986).

Possible mutations are transitions, transversions, insertions and deletions of at least one (1) base pair or nucleotide. Depending on the effect of the amino acid exchange caused by the mutation on the enzyme activity, "missense mutations" or "nonsense mutations" are referred to. Missense mutation leads to an exchange of a given amino acid in a protein for another, this being, in particular, a non-conservative amino acid exchange. The functional capacity or activity of the protein is impaired by this means and reduced to a value of 0 to 75%, 0 to 50%, 0 to 25%, 0 to 10% or 0 to 5%. Nonsense mutation leads to a stop codon in the coding region of the gene and therefore to a premature interruption in the translation. Insertions or deletions of at least one base pair in a gene lead to frame shift mutations, which lead to incorrect amino acids being incorporated or translation being interrupted prematurely. If a stop codon is formed in the coding region as a consequence of the mutation, this also leads to a premature termination of the translation. Deletions of at least one (1) or more codons typically also lead to a complete loss of the enzyme activity.

Instructions on generation of such mutations are prior art and can be found in known textbooks of genetics and molecular biology, such as e.g. the textbook by Knippers ("Molekulare Genetik [Molecular Genetics]", 6th edition, Georg Thieme Verlag, Stutt--gart, Germany, 1995), that by Winnacker ("Gene und Klone [Genes and Clones]", VCH Verlagsgesellschaft, Weinheim, Germany, 1990) or that by Hagemann ("Allgemeine Genetik [General Genetics]", Gustav Fischer Verlag, Stuttgart, 1986).

Suitable mutations in the genes can be incorporated into suitable strains by gene or allele replacement.

A common method is the method, described by Hamilton et al. (Journal of Bacteriology 171: 4617-4622 (1989)), of gene replacement with the aid of a conditionally replicating pSC101 derivative pMAK705- Other methods described in the prior art, such as, for example, that of Martinez-Morales et al. (Journal of Bacteriology 181: 7143-7148 (1999)) or that of Boyd et al. (Journal of Bacteriology 182: 842-847 (2000)), can likewise be used.

It is also possible to transfer mutations in the particular genes or mutations which affect expression of the particular genes or open reading frames into various strains by conjugation or transduction.

In addition to enhancement of the eno gene it may furthermore be advantageous for the production of L-threonine and L-lysine to eliminate undesirable side reactions (Nakayama: "Breeding of Amino Acid Producing Microorganisms", in: Overproduction of Microbial Products, Krumphanzl, Sikyta, Vanek (eds.), Academic Press, London, UK, 1982).

The microorganisms produced can be cultured in the batch process (batch culture), in the fed batch (feed process), in the repeated fed batch process (repetitive feed process) or in a continuous process (DE102004028859.3 or US5,763,230). A summary of known culture methods is described in the textbook by Chmi el (Bioprozesstechnik 1. Einführung in die Bioverfahrenstechnik (Gustav Fischer Verlag, Stuttgart, 1991)) or in the textbook by Storhas (Bioreaktoren und periphere Einrichtungen (Vieweg Verlag, Braunschweig/Wiesbaden, 1994)).

The culture medium to be used must meet the requirements of the particular strains in a suitable manner. Descriptions of culture media for various microorganisms are contained in the handbook "Manual of Methods for General Bacteriology" of the American Society for Bacteriology (Washington D.C., USA, 1981).

Sugars and carbohydrates, such as e.g. glucose, sucrose, lactose, fructose, maltose, molasses, starch and optionally cellulose, oils and fats, such as e.g. soya oil, sunflower oil, groundnut oil and coconut fat, fatty acids, such as e.g. palmitic acid, stearic acid and linoleic acid, alcohols, such as e.g. glycerol and ethanol, and organic acids, such as e.g. acetic acid, can be used as the source of carbon. These substances can be used individually or as a mixture.

Organic nitrogen-containing compounds, such as peptones, yeast extract, meat extract, malt extract, corn steep liquor, soya bean flour and urea, or inorganic compounds, such as ammonium sulfate, ammonium chloride, ammonium phosphate, ammonium carbonate and ammonium nitrate, can be used as the source of nitrogen. The sources of nitrogen can be used individually or as a mixture.

Phosphoric acid, potassium dihydrogen phosphate or dipotassium hydrogen phosphate or the corresponding sodium-containing salts can be used as the source of phosphorus. The culture medium must furthermore comprise salts of metals, such as e.g. magnesium sulfate or iron sulfate, which are necessary for growth. Finally, essential growth substances, such as amino acids and vitamins, can be employed in addition to the abovementioned substances. Suitable precursors can moreover be added to the culture medium. The starting substances mentioned can be added to the culture in the form of a single batch, or can be fed in during the culture in a suitable manner.

The fermentation is in general carried out at a pH of 5.5 to 9.0, in particular 6.0 to 8.0. Basic compounds, such as sodium hydroxide, potassium hydroxide, ammonia or aqueous ammonia, or acid compounds, such as phosphoric acid or sulfuric acid, can be employed in a suitable manner to control the pH of the culture. Antifoams, such as e.g. fatty acid polyglycol esters, can be employed to control the development of foam. Suitable substances having a selective action, e.g. antibiotics, can be added to the medium to maintain the stability of plasmids. To maintain aerobic conditions, oxygen or oxygen-containing gas mixtures, such as e.g. air, are introduced into the culture. The temperature of the culture is usually 25ºC to 45ºC, and preferably 30ºC to 40ºC. Culturing is continued until a maximum of L-amino acids or L-threonine has formed. This target is usually reached within 10 hours to 160 hours.

The analysis of L-amino acids can be carried out by anion exchange chromatography with subsequent ninhydrin derivatization, as described by Spackman et al. (Analytical Chemistry, 30: 1190-1206 (1958)) or it can be carried out by reversed phase HPLC, as described by Lindroth et al. (Analytical Chemistry 51: 1167-1174 (1979)).

The process according to the invention is used for the fermentative preparation of L-threonine and L-lysine, in particular L-threonine.

The following microorganism has been deposited at the Deutsche Sammlung für Mikroorganismen und Zellkulturen (DSMZ = German Collection of Microorganisms and Cell Cultures, Braunschweig, Germany) in accordance with the Budapest Treaty:
- Escherichia coli strain E. coli MG442 as DSM 16574.

The present invention is explained in more detail in the following with the aid of embodiment examples.

The minimal (M9) and complete media (LB) for Escherichia coli used are described by J.H. Miller (A Short Course in Bacterial Genetics (1992), Cold Spring Harbor Laboratory Press). The isolation of plasmid DNA from Escherichia coli and all techniques of restriction, ligation, Klenow and alkaline phosphatase treatment are carried out by the method of Sambrook et al. (Molecular Cloning - A Laboratory Manual (1989) Cold Spring Harbor Laboratory Press). Unless described otherwise, the transformation of Escherichia coli is carried out by the method of Chung et al. (Proceedings of the National Academy of Sciences of the United States of America 86: 2172-2175 (1989)).

The incubation temperature for the preparation of strains and transformants is 37ºC.

### Example 1

### Construction of the expression plasmid pTrc99Aeno

The eno gene from E. coli K12 is amplified using the polymerase chain reaction (PCR) and synthetic oligonucleotides. Starting from the nucleotide sequence of the eno gene in E. coli K12 MG1655 (Accession Number AE000361, Blattner et al. (Science 277: 1453-1474 (1997)), PCR primers are synthesized (MWG Biotech, Ebersberg, Germany). The primers contain sequences for restriction enzymes which are marked by underlining in the nucleotide sequence shown below. The primer eno1 contains the restriction cleavage site for XabI, the primer eno2 contains that for HindIII.
enol:
   5' - GTTTGTCTAGAGTTTCAGTTTAACTAGTGAC - 3' (SEQ ID No. 1)
eno2 :
   5' - CCGGAGGCTGGCAAGCTTAAATCAG - 3' (SEQ ID No. 2)

The chromosomal E. coli K12 MG1655 DNA employed for the PCR is isolated according to the manufacturers instructions with "Qiagen Genomic-tips 100/G" (QIAGEN, Hilden, Germany). A DNA fragment approx. 1,381 bp in size can be amplified with the specific primers under standard PCR conditions (Innis et al. (1990) PCR Protocols. A Guide to Methods and Applications, Academic Press) with Vent-DNA polymerase (New England BioLabs, Frankfurt, Germany) (SEQ ID No. 3).

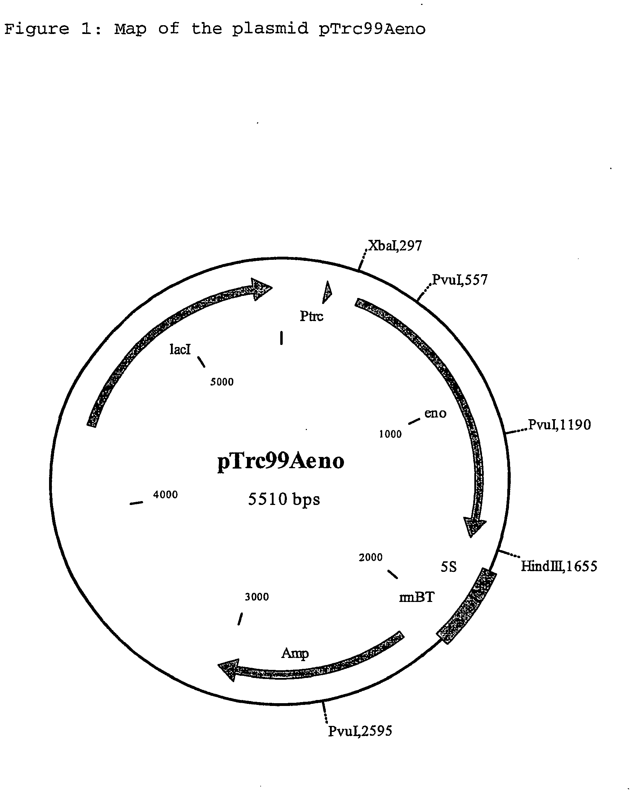

The amplified eno fragment is restricted with the restriction enzymes HindIII and XbaI and after purification (Purification Kit, QIAGEN, Hilden, Germany) checked in a 0.8% agarose gel. The vector pTrc99A (Pharmacia Biotech, Uppsala, Sweden) is cleaved with the enzymes HindIII and XbaI and ligation is carried out with the restricted eno fragment. The E. coli strain TOP10 One Shot (TOPO TA Cloning Kit, Invitrogen, Groningen, The Netherlands) is transformed with the ligation batch and plasmid-carrying cells are selected on LB agar, to which 50 µg/ml ampicillin is added. Successful cloning can be demonstrated after plasmid DNA isolation by control cleavage with the enzymes HindIII/XbaI and PvuI. The plasmid is called pTrc99Aeno (figure 1).

### Example 2

### Preparation of L-threonine with the strain MG442/pTrc99Aeno

The L-threonine-producing E. coli strain MG442 is described in the patent specification US-A-4,278,765 and deposited as CMIM B-1628 at the Russian National Collection for Industrial Microorganisms (VKPM, Moscow, Russia) and as DSM 16574 at the Deutsche Sammlung für Mikroorganismen und Zellkulturen (DSMZ = German Collection of Microorganisms and Cell Cultures, Braunschweig, Germany) in accordance with the Budapest Treaty.

The strain MG442 is transformed with the expression plasmid pTrc99Aeno described in example 1 and with the vector pTrc99A and plasmid-carrying cells are selected on LB agar with 50 µg/ml ampicillin. Successful transformations can be demonstrated after plasmid DNA isolation by control cleavages with the enzymes HindIII/XbaI. The strains MG442/pTrc99Aeno and MG442/pTrc99A are formed in this manner. Selected individual colonies are then multiplied further on minimal medium with the following- composition: 3.5 g/l Na₂HPO₄*2H₂O, 1.5 g/l KH₂PO₄, 1 g/l NH₄Cl, 0.1 g/l MgSO₄*7H₂O, 2 g/l glucose, 20 g/l agar, 50 mg/l ampicillin. The formation of L-threonine is checked in batch cultures of 10 ml contained in 100 ml conical flasks. For this, 10 ml of preculture medium of the following composition: 2 g/l yeast extract, 10 g/l (NH₄)₂SO₄, 1 g/l KH₂PO₄, 0.5 g/l MgSO₄*7H₂O, 15 g/l CaCO₃, 20 g/l glucose, 50 mg/l ampicillin are inoculated and the batch is incubated for 16 hours at 37ºC and 180 rpm on an ESR incubator from Kühner AG (Birsfelden, Switzerland).

250 µl portions of this preculture are trans inoculated into 10 ml of production medium (25 g/l (NH₄)₂O₄, 2 g/l KH₂PO₄, 1 g/l MgSO₄*7H₂O, 0.03 g/l FeSO₄*7H₂O, 0.018 g/l MnSO₄*1H₂O, 30 g/l CaCO₃, 20 g/l glucose, 50 mg/l ampicillin) and the batch is incubated for 48 hours at 37ºC. For complete induction of the expression of the eno gene, 100 mg/l isopropyl β-D-thiogalactopyranoside (IPTG) are added in parallel batches. The formation of L-threonine by the starting strain MG442 is investigated in the same manner, but no addition of ampicillin to the medium takes place. After the incubation the optical density (OD) of the
culture suspension is determined with an LP2W photometer from Dr. Lange (Düsseldorf, Germany) at a measurement wavelength of 660 nm.

The concentration of L-threonine formed is then determined in the sterile-filtered culture supernatant with an amino acid analyzer from Eppendorf-BioTronik (Hamburg, Germany) by ion exchange chromatography and post-column reaction with ninhydrin detection.

The result of the experiment is shown in Table 1.

**Table 1**

| Strain | Additives | OD (660 nm) | L-Threonine g/l |
|---|---|---|---|
| MG442 | - | 5.6 | 1.4 |
| MG442/pTrc99A | - | 3.8 | 1.3 |
| MG442/pTrc99Aeno | - | 2.6 | 1.7 |
| MG442/pTrc99Aeno | IPTG | 5.1 | 2.6 |

### Brief description of the Figure:

### Figure 1: Map of the plasmid pTrc99Aeno containing the eno gene

The length data are to be understood as approx. data. The abbreviations and designations used have the following meaning:
- Amp: ampicillin resistance gene
- lacI: gene for the repressor protein of the trc promoter
- Ptrc: trc promoter region, IPTG-inducible
- eno: coding region of the eno gene
- 5S: 5S rRNA region
- rrnBT: rRNA terminator region
The abbreviations for the restriction enzymes have the following meaning
- HindIII: restriction endonuclease from Haemophilus influenzae R_{C}
- PvuI: restriction endonuclease from Proteus vulgaris
- XbaI: restriction endonuclease from Xanthomonas campestris

## Claims

1. Process for the preparation of L-threonine or L-lysine by fermentation of recombinant microorganisms of the Enterobacteriaceae family, wherein
a) the microorganisms, which produce the desired L-amino acid and in which the eno gene or nucleotide sequences or alleles which code for a protein with the activity of an enolase are enhanced, are cultured in a medium under conditions under which the desired L-amino acid is concentrated in the medium or in the cells, and
b) the desired L-amino acid is isolated, constituents of the fermentation broth and/or the biomass in its entirety or portions (≥ 0 to 100%) thereof remaining in the product which has been isolated or being completely removed,
wherein enhancement describes the increased intracellular activity or concentration of said protein by at least 10% based on that of the wild-type protein or on that of the parent strain.

2. Process according to claim 1 in which a polynucleotide which codes for a polypeptide of which the amino acid sequence is identical to the extent of at least 90% to an amino acid sequence chosen from the group consisting of SEQ ID No. 4, SEQ ID No. 6, and SEQ ID No. 8 is enhanced.

3. Process according to claim 2, wherein the microorganisms contain an over-expressed or enhanced polynucleotide corresponding to the eno gene chosen from the group consisting of:
a) polynucleotide with the nucleotide sequence from SEQ ID No. 3, SEQ ID No. 5 or SEQ ID No. 7;
b) polynucleotide with a nucleotide sequence which corresponds to SEQ ID No. 3, SEQ ID No. 5 or SEQ ID No. 7 within the context of degeneration of the genetic code;
c) polynucleotide sequence with a sequence which hybridizes under stringent conditions with the sequence complementary to SEQ ID No. 3, SEQ ID No. 5 or SEQ ID No. 7;
d) polynucleotide with a sequence SEQ ID No. 3, SEQ ID No. 5 or SEQ ID No. 7 which contains neutral-function sense mutants.

4. Process according to claim 2, wherein the polypeptide has an amino acid sequence which is identical to the extent of at least 95% to one of the sequences chosen from the group consisting of SEQ ID No. 4, SEQ ID No. 6 and SEQ ID No. 8.

5. Process according to claim 2, wherein the polypeptide has the amino acid sequence which is 100% identical to that from SEQ ID No. 4, SEQ ID No. 6 or SEQ ID No. 8.

6. Process according to claims 1 to 5, wherein the microorganisms are produced by transformation, transduction or conjugation, or a combination of these methods, with a vector which contains the eno gene, an allele of this gene or parts thereof and/or a promoter.

7. Process according to claims 1 or 6, in which the number of copies of the eno gene or of the alleles is increased by at least 1.

8. Process according to claim 7, wherein the increase in the number of copies of the eno gene by at least 1 is effected by integration of the ORF or the alleles into the chromosome of the microorganisms.

9. Process according to claim 7, wherein the increase in the number of copies of the eno gene by at least 1 is achieved by a vector which replicates extrachromosomally.

10. Process according to claims 1 or 2, wherein, to achieve the enhancement,
a) the promoter and regulation region or the ribosome-binding site upstream of the eno gene is mutated, or
b) expression cassettes or promoters are incorporated upstream of the eno gene.

11. Process according to claims 1 or 2, wherein the eno gene is under the control of a promoter which enhances expression of the gene.

12. Process according to claims 1 to 11, wherein the concentration or activity of the eno gene product is increased by overexpression of the eno gene or nucleotide sequences or alleles which code for the gene product thereof.

13. Process according to claims 1 to 12, wherein the microorganisms are chosen from the genus Escherichia.

14. Process according to claims 1 to 13, wherein the microorganisms are chosen from the species Escherichia coli.

15. Process according to claims 1 to 14, wherein, for the preparation of L-threonine, microorganisms of the Enterobacteriaceae family are fermented in which additionally at the same time one or more of the genes chosen from the group consisting of:
15.1 at least one gene of the thrABC operon which codes for aspartate kinase, homoserine dehydrogenase, homoserine kinase and threonine synthase,
15.2 the pyc gene of Corynebacterium glutamicum which codes for pyruvate carboxylase,
15.3 the pps gene which codes for phosphoenol pyruvate synthase,
15.4 the ppc gene which codes for phosphoenol pyruvate carboxylase,
15.5 the pntA and pntB genes which code for subunits of pyridine transhydrogenase,
15.6 the rhtB gene which codes for the protein which imparts homoserine resistance,
15.7 the rhtC gene which codes for the protein which imparts threonine resistance,
15.8 the thrE gene of Corynebacterium which codes for the threonine export carrier protein,
15.9 the gdhA gene which codes for glutamate dehydrogenase,
15.10 the pgm gene which codes for phosphoglucomutase,
15.11 the fba gene which codes for fructose biphosphate aldolase,
15.12 the ptsH gene which codes for the phosphohistidine protein hexose phosphotransferase,
15.13 the ptsI gene which codes for enzyme I of the phosphotransferase system,
15.14 the crr gene which codes for the glucose-specific IIA component,
15.15 the ptsG gene which codes for the glucose-specific IIBC component,
15.16 the lrp gene which codes for the regulator of the leucine regulon,
15.17 the fadR which codes for the regulator of the fad regulon,
15.18 the iclR gene which codes for the regulator of central intermediate metabolism,
15.19 the ahpC gene which codes for the small sub-unit of alkyl hydroperoxide reductase,
15.20 the ahpF gene which codes for the large sub-unit of alkyl hydroperoxide reductase,
15.21 the cysK gene which codes for cysteine synthase A,
15.22 the cysB gene which codes for the regulator of the cys regulon,
15.23 the cysJ gene which codes for the flavoprotein of NADPH sulfite reductase,
15.24 the cysI gene which codes for the haemoprotein of NADPH sulfite reductase,
15.25 the cysH gene which codes for adenylyl sulfate reductase,
15.26 the rseA gene which codes for a membrane protein with anti-sigmaE activity,
15.27 the rseC gene which codes for a global regulator of the sigmaE factor,
15.28 the sucA gene which codes for the decarboxylase sub-unit of 2-ketoglutarate dehydrogenase,
15.29 the sucB gene which codes for the dihydrolipoyltranssuccinase E2 sub-unit of 2-ketoglutarate dehydrogenase,
15.30 the sucC gene which codes for the β-sub-unit of succinyl-CoA synthetase,
15.31 the sucD gene which codes for the α-sub-unit of succinyl-CoA synthetase,
15.32 the aceE gene which codes for the E1 component of the pyruvate dehydrogenase complex,
15.33 the aceF gene which codes for the E2 component of the pyruvate dehydrogenase complex,
15.34 the rseB gene which codes for the regulator of sigmaE factor activity,
15.35 the gene product of the open reading frame (ORF) yodA of Escherichia coli, and
15.36 the gene product of the open reading frame (ORF) yaaU of Escherichia coli
is or are enhanced, in particular over-expressed.

16. Process according to claims 1 to 15, wherein, for the preparation of L-threonine, microorganisms of the Enterobacteriaceae family are fermented in which additionally at the same time one or more of the genes chosen from the group consisting of:
16.1 the tdh gene which codes for threonine dehydrogenase,
16.2 the mdh gene which codes for malate dehydrogenase,
16.3 the gene product of the open reading frame (ORF) yjfA of Escherichia coli,
16.4 the gene product of the open reading frame (ORF) ytfP of Escherichia coli,
16.5 the pckA gene which codes for phosphoenol pyruvate carboxykinase,
16.6 the poxB gene which codes for pyruvate oxidase,
16.7 the dgsA gene which codes for the DgsA regulator of the phosphotransferase system,
16.8 the fruR gene which codes for the fructose repressor,
16.9 the rpoS gene which codes for the sigma³⁸ factor, and
16.10 the aspA gene which codes for aspartate ammonium lyase
is or are attenuated, in particular eliminated or reduced in expression.

## Patentansprüche

1. Verfahren zur Herstellung von L-Threonin oder L-Lysin durch Fermentation rekombinanter Mikroorganismen der Familie Enterobacteriaceae, wobei man
a) die Mikroorganismen, die die gewünschte L-Aminosäure produzieren und in denen das eno-Gen oder Nukleotidsequenzen oder Allele, die für ein Protein mit der Aktivität einer Enolase codieren, verstärkt sind, in einem Medium unter Bedingungen, bei denen die gewünschte L-Aminosäure in dem Medium oder in den Zellen angereichert wird, kultiviert und
b) die gewünschte L-Aminosäure isoliert, wobei Bestandteile der Fermentationsbrühe und/oder die komplette Biomasse oder Anteile (≥0 bis 100%) davon in dem Produkt, das isoliert wurde, verbleiben oder vollständig abgetrennt werden,
wobei Verstärkung die um wenigstens 10% erhöhte intrazelluläre Aktivität oder Konzentration des Proteins, bezogen auf die des Wildtyp-Proteins oder auf die des Elternstamms, beschreibt.

2. Verfahren nach Anspruch 1, bei dem ein Polynukleotid, das für ein Polypeptid codiert, dessen Aminosäuresequenz zu wenigstens 90% mit einer aus der aus SEQ ID Nr. 4, SEQ ID Nr. 6 und SEQ ID Nr. 8 bestehenden Gruppe gewählten Aminosäuresequenz identisch ist, verstärkt ist.

3. Verfahren nach Anspruch 2, wobei die Mikroorganismen ein überexprimiertes oder verstärktes Polynukleotid enthalten, das dem aus der folgenden Gruppe gewählten eno-Gen entspricht:
a) Polynukleotid mit der Nukleotidsequenz aus SEQ ID Nr. 3, SEQ ID Nr. 5 oder SEQ ID Nr. 7;
b) Polynukleotid mit einer Nukleotidsequenz, die im Rahmen der Degeneriertheit des genetischen Codes SEQ ID Nr. 3, SEQ ID Nr. 5 oder SEQ ID Nr. 7 entspricht;
c) Polynukleotidsequenz mit einer Sequenz, die unter stringenten Bedingungen mit der zu SEQ ID Nr. 3, SEQ ID Nr. 5 oder SEQ ID Nr. 7 komplementären Sequenz hybridisiert;
d) Polynukleotid mit einer Sequenz SEQ ID Nr. 3, SEQ ID Nr. 5 oder SEQ ID Nr. 7, die Sense-Mutanten mit neutraler Funktion enthält.

4. Verfahren nach Anspruch 2, wobei das Polypeptid eine Aminosäuresequenz aufweist, die zu wenigstens 95% mit einer der aus der aus SEQ ID Nr. 4, SEQ ID Nr. 6 und SEQ ID Nr. 8 bestehenden Gruppe gewählten Sequenzen identisch ist.

5. Verfahren nach Anspruch 2, wobei das Polypeptid die Aminosäuresequenz aufweist, die mit der aus SEQ ID Nr. 4, SEQ ID Nr. 6 oder SEQ ID Nr. 8 100% identisch ist.

6. Verfahren nach Anspruch 1 bis 5, wobei die Mikroorganismen mittels Transformation, Transduktion oder Konjugation oder einer Kombination dieser Methoden mit einem Vektor, der das eno-Gen, ein Allel dieses Gens oder Teile davon und/oder einen Promotor enthält, erzeugt werden.

7. Verfahren nach Anspruch 1 bis 6, bei dem die Kopienzahl des eno-Gens oder der Allele um wenigstens 1 erhöht ist.

8. Verfahren nach Anspruch 7, wobei die Erhöhung der Kopienzahl des eno-Gens um wenigstens 1 durch Integration des ORF oder der Allele in das Chromosom der Mikroorganismen bewirkt wird.

9. Verfahren nach Anspruch 7, wobei die Erhöhung der Kopienzahl des eno-Gens um wenigstens 1 mit einem Vektor, der extrachromosomal repliziert, erreicht wird.

10. Verfahren nach Anspruch 1 oder 2, wobei zur Erreichung der Verstärkung
a) der Promotor- und Regulationsbereich oder die Ribosomenbindungsstelle stromaufwärts des eno-Gens mutiert wird oder
b) Expressionskassetten oder Promotoren stromaufwärts des eno-Gens eingebaut werden.

11. Verfahren nach Anspruch 1 oder 2, wobei das eno-Gen unter der Kontrolle eines Promotors steht, der die Expression des Gens verstärkt.

12. Verfahren nach Anspruch 1 bis 11, wobei die Konzentration oder Aktivität des eno-Genprodukts durch Überexpression des eno-Gens oder von Nukleotidsequenzen oder Allelen, die für das Genprodukt davon codieren, erhöht wird.

13. Verfahren nach Anspruch 1 bis 12, wobei die Mikroorganismen aus der Gattung Escherichia gewählt sind.

14. Verfahren nach Anspruch 1 bis 13, wobei die Mikroorganismen aus der Spezies Escherichia coli gewählt sind.

15. Verfahren nach Anspruch 1 bis 14, wobei zur Herstellung von L-Threonin Mikroorganismen der Familie Enterobacteriaceae fermentiert werden, in denen zusätzlich gleichzeitig eines oder mehrere der aus der Gruppe:
15.1 wenigstens ein Gen des thrABC-Operons, das für Aspartat-Kinase, Homoserin-Dehydrogenase, Homoserin-Kinase und Threonin-Synthase codiert,
15.2 das pyc-Gen aus Corynebacterium glutamicum, das für Pyruvat-Carboxylase codiert,
15.3 das pps-Gen, das für Phosphoenolpyruvat-Synthase codiert,
15.4 das ppc-Gen, das für Phosphoenolpyruvat-Carboxylase codiert,
15.5 die Gene pntA und pntB, die für Untereinheiten der Pyridin-Transhydrogenase codieren,
15.6 das rhtB-Gen, das für das Homoserinresistenz verleihende Protein codiert,
15.7 das rhtC-Gen, das für das Threoninresistenz verleihende Protein codiert,
15.8 das thrE-Gen aus Corynebacterium, das für das Threoninexport-Carrier-Protein codiert,
15.9 das gdhA-Gen, das für Glutamat-Dehydrogenase codiert,
15.10 das pgm-Gen, das für Phosphoglucomutase codiert,
15.11 das fba-Gen, das für Fructosebiphosphat-Aldolase codiert,
15.12 das ptsH-Gen, das für die Phosphohistidinprotein-Hexose-Phosphotransferase codiert,
15.13 das ptsI-Gen, das für Enzym I des Phosphotransferase-Systems codiert,
15.14 das crr-Gen, das für die glucosespezifische IIA-Komponente codiert,
15.15 das ptsG-Gen, das für die glucosespezifische IIBC-Komponente codiert,
15.16 das lrp-Gen, das für den Regulator des Leucin-Regulons codiert,
15.17 das fadR, das für den Regulator des fad-Regulons codiert,
15.18 das iclR-Gen, das für den Regulator des zentralen Intermediärstoffwechsels codiert,
15.19 das ahpC-Gen, das für die kleine Untereinheit der Alkylhydroperoxid-Reduktase codiert,
15.20 das ahpF-Gen, das für die große Untereinheit der Alkylhydroperoxid-Reduktase codiert,
15.21 das cysK-Gen, das für Cystein-Synthase A codiert,
15.22 das cysB-Gen, das für den Regulator des cys-Regulons codiert,
15.23 das cysJ-Gen, das für das Flavoprotein der NADPH-Sulfit-Reduktase codiert,
15.24 das cysI-Gen, das für das Hämoprotein der NADPH-Sulfit-Reduktase codiert,
15.25 das cysH-Gen, das für Adenylylsulfat-Reduktase codiert,
15.26 das rseA-Gen, das für ein Membranprotein mit Anti-sigmaE-Aktivität codiert,
15.27 das rseC-Gen, das für einen globalen Regulator des sigmaE-Faktors codiert,
15.28 das sucA-Gen, das für die Decarboxylase-Untereinheit der 2-Ketoglutarat-Dehydrogenase codiert,
15.29 das sucB-Gen, das für die Dihydrolipoyltranssuccinase-E2-Untereinheit der 2-Ketoglutarat-Dehydrogenase codiert,
15.30 das sucC-Gen, das für die β-Untereinheit der Succinyl-CoA-Synthetase codiert,
15.31 das sucD-Gen, das für die a-Untereinheit der Succinyl-CoA-Synthetase codiert,
15.32 das aceE-Gen, das für die El-Komponente des Pyruvat-Dehydrogenase-Komplexes codiert,
15.33 das aceF-Gen, das für die E2-Komponente des Pyruvat-Dehydrogenase-Komplexes codiert,
15.34 das rseB-Gen, das für den Regulator von sigmaE-Faktor-Aktivität codiert,
15.35 das Genprodukt des offenen Leserasters (ORF) yodA aus Escherichia coli und
15.36 das Genprodukt des offenen Leserasters (ORF) yaaU aus Escherichia coli
gewählten Gene verstärkt, insbesondere überexprimiert, ist bzw. sind.

16. Verfahren nach Anspruch 1 bis 15, wobei zur Herstellung von L-Threonin Mikroorganismen der Familie Enterobacteriaceae fermentiert werden, in denen zusätzlich gleichzeitig eines oder mehrere der aus der Gruppe:
16.1 das tdh-Gen, das für Threonin-Dehydrogenase codiert,
16.2 das mdh-Gen, das für Malat-Dehydrogenase codiert,
16.3 das Genprodukt des offenen Leserasters (ORF) yjfA aus Escherichia coli
16.4 das Genprodukt des offenen Leserasters (ORF) ytfP aus Escherichia coli
16.5 das pckA-Gen, das für Phosphoenolpyruvat-Carboxykinase codiert,
16.6 das poxB-Gen, das für Pyruvat-Oxidase codiert,
16.7 das dgsA-Gen, das für den DgsA-Regulator des Phosphotransferase-Systems codiert,
16.8 das fruR-Gen, das für den Fructose-Repressor codiert,
16.9 das rpoS-Gen, das für den sigma³⁸-Faktor codiert, und
16.10 das aspA-Gen, das für Aspartat-Ammonium-Lyase codiert,
gewählten Gene abgeschwächt ist bzw. sind, insbesondere ausgeschaltet ist bzw. sind oder eine verminderte Expression aufweist bzw. aufweisen.

## Revendications

1. Procédé de préparation de L-thréonine ou de L-lysine par fermentation de micro-organismes recombinants de la famille des Enterobacteriaceae, dans lequel
a) les micro-organismes qui produisent l'acide L-aminé souhaité et dans lesquels le gène eno ou des séquences nucléotidiques ou des allèles qui codent pour une protéine ayant l'activité d'une énolase sont amplifiés, sont cultivés dans un milieu dans des conditions dans lesquelles l'acide L-aminé souhaité est concentré dans le milieu ou dans les cellules, et
b) l'acide L-aminé souhaité est isolé, les constituants du bouillon de fermentation et/ou de la biomasse dans leur totalité ou dans des parties (≥ 0 à 100 %) de ces derniers, restant dans le produit qui a été isolé ou qui est en cours d'élimination complète,
dans lequel l'amplification décrit l'augmentation de l'activité intracellulaire ou de la concentration de ladite protéine d'au moins 10 % par rapport à celle de la protéine de type sauvage ou à celle de la souche parentale.

2. Procédé selon la revendication 1, dans lequel un polynucléotide qui code pour un polypeptide dont la séquence d'acides aminés a une identité d'au moins 90 % avec une séquence d'acides aminés choisie dans le groupe consistant en SEQ ID N° 4, SEQ ID N° 6 et SEQ ID N° 8 est amplifié.

3. Procédé selon la revendication 2, dans lequel les micro-organismes contiennent un polynucléotide surexprimé ou amplifié, correspondant au gène eno, choisi dans le groupe consistant en :
a) un polynucléotide ayant la séquence nucléotidique choisie parmi SEQ ID N° 3, SEQ ID N° 5 ou SEQ ID N° 7 ;
b) un polynucléotide ayant une séquence nucléotidique qui correspond à SEQ ID N° 3, SEQ ID N° 5 ou SEQ ID N° 7 dans le contexte de la dégénérescence du code génétique ;
c) une séquence polynucléotidique ayant une séquence qui s'hybride dans des conditions stringentes avec la séquence complémentaire de SEQ ID N° 3, SEQ ID N° 5 ou SEQ ID N° 7 ;
d) un polynucléotide ayant une séquence SEQ ID N° 3, SEQ ID N° 5 ou SEQ ID N° 7, qui contient des mutants sens à fonction neutre.

4. Procédé selon la revendication 2, dans lequel le polypeptide a une séquence d'acides aminés qui a une identité d'au moins 95 % avec l'une des séquences choisies dans le groupe consistant en SEQ ID N° 4, SEQ ID N° 6 et SEQ ID N° 8.

5. Procédé selon la revendication 2, dans lequel le polypeptide a la séquence d'acides aminés qui a une identité de 100 % avec la séquence SEQ ID N° 4, SEQ ID N° 6 ou SEQ ID N° 8.

6. Procédé selon les revendications 1 à 5, dans lequel les micro-organismes sont produits par transformation, transduction ou conjugaison, ou une combinaison de ces procédés, avec un vecteur qui contient un gène eno, un allèle de ce gène ou des parties de ce dernier et/ou un promoteur.

7. Procédé selon la revendication 1 ou 6, dans lequel le nombre de copies du gène eno ou des allèles est augmenté d'au moins 1.

8. Procédé selon la revendication 7, dans lequel l'augmentation du nombre de copies du gène eno d'au moins 1 est réalisée par intégration de l'ORF ou des allèles dans le chromosome des micro-organismes.

9. Procédé selon la revendication 7, dans lequel l'augmentation du nombre de copies du gène eno d'au moins 1 est réalisée par un vecteur qui se réplique d'une manière extra-chromosomique.

10. Procédé selon la revendication 1 ou 2, dans lequel, pour réaliser l'amplification,
a) la région promotrice et la région de régulation ou le site de liaison aux ribosomes, en amont du gène eno, sont mutés, ou
b) des cassettes d'expression ou des promoteurs sont incorporés en amont du gène eno.

11. Procédé selon la revendication 1 ou 2, dans lequel le gène eno se trouve sous le contrôle d'un promoteur qui amplifie l'expression du gène.

12. Procédé selon les revendications 1 à 11, dans lequel la concentration ou l'activité du produit du gène eno est augmentée par surexpression du gène eno ou des séquences nucléotidiques ou des allèles qui codent pour leur produit génique.

13. Procédé selon les revendications 1 à 12, dans lequel les micro-organismes sont choisis dans le genre Escherichia.

14. Procédé selon les revendications 1 à 13, dans lequel les micro-organismes sont choisis dans l'espèce Escherichia coli.

15. Procédé selon les revendications 1 à 14, dans lequel, pour la préparation de L-thréonine, les micro-organismes de la famille des Enterobacteriaceae sont fermentés, dans lesquels on amplifie, en particulier on surexprime en outre, simultanément, un ou plusieurs des gènes choisis dans le groupe consistant en :
15.1 au moins un gène de l'opéron thrABC qui code pour l'aspartate kinase, l'homosérine déshydrogénase, l'homosérine kinase et la thréonine synthase,
15.2 le gène pyc de Corynebacterium glutamicum, qui code pour la pyruvate carboxylase,
15.3 le gène pps, qui code pour la phosphoénol pyruvate synthase,
15.4 le gène ppc, qui code pour la phosphoénol pyruvate carboxylase,
15.5 les gènes pntA et pntB, qui codent pour des sous unités de la pyridine transhydrogénase,
15.6 le gène rhtB, qui code pour la protéine qui confère une résistance à l'homosérine,
15.7 le gène rhtC, qui code pour la protéine qui confère une résistance à la thréonine,
15.8 le gène thrE de Corynebacterium, qui code pour la protéine porteuse d'exportation de la thréonine,
15.9 le gène gdhA, qui code pour la glutamate déshydrogénase,
15.10 le gène pgm, qui code pour la phosphoglucomutase,
15.11 le gène fba, qui code pour la fructose biphosphate aldolase,
15.12 le gène ptsH, qui code pour la phosphohistidine protéine hexose phosphotransférase,
15.13 le gène ptsI, qui code pour l'enzyme I du système phosphotransférase,
15.14 le gène crr, qui code pour le composant IIA spécifique de glucose,
15.15 le gène ptsG, qui code pour le composant IIBC spécifique de glucose,
15.16 le gène lrp, qui code pour le régulateur du régulon de la leucine,
15.17 le gène fadR, qui code pour le régulateur du régulon fad,
15.18 le gène iclR, qui code pour le régulateur du métabolisme intermédiaire central,
15.19 le gène ahpC, qui code pour la petite sous-unité de l'alkyl hydroperoxyde réductase,
15.20 le gène ahpF, qui code pour la grande sous-unité de l'alkyl hydroperoxyde réductase,
15.21 le gène cysK, qui code pour la cystéine synthase A,
15.22 le gène cysB, qui code pour le régulateur du régulon cys,
15.23 le gène cysJ, qui code pour la flavoprotéine de la NADPH sulfite réductase,
15.24 le gène cysI, qui code pour l'hémoprotéine de la NADPH sulfite réductase,
15.25 le gène cysH, qui code pour l'adénylyl sulfate réductase,
15.26 le gène rseA, qui code pour une protéine membranaire ayant une activité anti-sigmaE,
15.27 le gène rseC, qui code pour un régulateur global du facteur sigmaE,
15.28 le gène sucA, qui code pour la sous-unité décarboxylase de la 2-kétoglutarate déshydrogénase, 15.29 le gène sucB, qui code pour la sous-unité dihydrolipoyltranssuccinase E2 de la 2-kétoglutarate déshydrogénase,
15.30 le gène sucC, qui code pour la sous-unité β de la succinyl CoA synthétase,
15.31 Le gène sucD, qui code pour la sous-unité α de la succinyl CoA synthétase,
15.32 le gène aceE, qui code pour le composant E1 du complexe pyruvate déshydrogénase,
15.33 le gène aceF, qui code pour le composant E2 du complexe pyruvate déshydrogénase,
15.34 le gène rseB, qui code pour le régulateur de l'activité du facteur sigmaE,
15.35 le produit génique du cadre ouvert de lecture (ORF) yodA d'Escherichia coli, et
15.36 le produit génique du cadre ouvert de lecture (ORF) yaaU d'Escherichia coli.

16. Procédé selon les revendications 1 à 15 dans lequel, pour la préparation de L-thréonine, des micro-organismes de la famille des Enterobacteriaceae sont fermentés, dans lesquels on atténue, en particulier on élimine ou on en réduit l'expression, additionnellement et simultanément un ou plusieurs des gènes choisis dans le groupe consistant en :
16.1 le gène tdh, qui code pour la thréonine déshydrogénase,
16.2 le gène mdh, qui code pour la malate déshydrogénase,
16.3 le produit génique du cadre ouvert de lecture (ORF) yjfA d'Escherichia coli,
16.4 le produit génique du cadre ouvert de lecture (ORF) ytfP d'Escherichia coli,
16.5 le gène pckA, qui code pour la phosphoénol pyruvate carboxykinase,
16.6 le gène poxB, qui code pour la pyruvate oxydase,
16.7 le gène dgsA, qui code pour le régulateur DgsA du système phosphotransférase,
16.8 le gène fruR, qui code pour le répresseur du fructose,
16.9 le gène rpoS, qui code pour le facteur sigma³⁸, et
16.10 le gène aspA, qui code pour l'aspartate ammonium lyase.
